# FASCICULE DE BREVET EUROPEEN

(11) **EP 1 814 905 B1**
(45) Date de publication et mention de la délivrance du brevet: **17.04.2013**
(21) Numéro de dépôt: 05817457.4
(22) Date de dépôt: 14.11.2005
(51) Int. Cl.: C07K 14/435, C07K 19/00, A61K 38/17, A61K 47/42, A61K 47/48, C12N 15/11

(54) **PEPTIDES DERIVES DE LA MAUROCALCINE UTILISABLES COMME VECTEURS POUR L'ADRESSAGE INTRACELLULAIRE DE MOLECULES D'INTERET**
VON MAUROCALCIN ABGELEITETE PEPTIDE, DIE ALS VEKTOREN FÜR DAS INTRAZELLULÄRE AUFFINDEN VON INTERESSIERENDEN MOLEKÜLEN VERWENDET WERDEN
PEPTIDES DERIVED FROM MAUROCALCINE USED AS VECTORS FOR INTRACELLULAR ADDRESSING OF MOLECULES OF INTEREST

(30) Priorité: 12.11.2004 FR 0412045
(43) Date de publication de la demande: 08.08.2007
(73) Titulaire: Commissariat à l'Énergie Atomique et aux Énergies Alternatives, 75015 Paris (FR); INSTITUT NATIONAL DE LA SANTE ET DE LA RECHERCHE MEDICALE (INSERM), 75013 Paris (FR)
(72) Inventeur: RONJAT, Michel, F-38410 Vaulnaveys le Haut (FR); DE WAARD, Michel, F-38380 Saint Christophe sur Guiers (FR)
(74) Mandataire: Leblois-Préhaud, Hélène Marthe Georgette
(86) Numéro de dépôt international: PCT/FR2005/002817
(87) Numéro de publication internationale: WO 2006/051224

(56) Documents cités:
- WO-A-00/15655
- WO-A-01/64724
- WO-A-03/106491
- WO-A2-03/106615
- US-A- 6 051 429
- MCKENZIE D L ET AL: "A potent new class of reductively activated peptide gene delivery agents" JOURNAL OF BIOLOGICAL CHEMISTRY, AMERICAN SOCIETY OF BIOLOCHEMICAL BIOLOGISTS, BIRMINGHAM,, US, vol. 275, no. 14, 7 avril 2000 (2000-04-07), pages 9970-9977, XP002238140 ISSN: 0021-9258
- YONEDA Y ET AL.: "Synthetic Peptides Containing a Region of SV 40 Large T-Antigen Involved in Nuclear Localization Direct the Transport of Proteins into the Nucleus" EXPERIMENTAL CELL RESEARCH, vol. 170, no. 2, 1987, pages 439-452, XP008062743 ISSN: 0014-4827
- TABOR AB ET AL.: "Fluorescently labelled peptides as tools for probing the structure and function of a non-viral gene delivery vector" JOURNAL OF PEPTIDE SCIENCE, vol. 8, no. Suppl., 2002, page S154, XP008062746
- PARK Y ET AL.: "Synthesis of Sulfhydryl Cross-Linking Poly(Ethylene Glycol)-Peptides and Glycopeptides as Carriers for Gene Delivery" BIOCONJUGATE CHEMISTRY, vol. 13, 2002, pages 232-239, XP002335023
- KWOK KY ET AL.: "In Vivo Gene Transfer Using Sulfhydryl Cross-Linked PEG-Peptide/Glycopeptide DNA-Co-Condensates" JOURNAL OF PHARMACEUTICAL SCIENCES, vol. 92, no. 6, juin 2003 (2003-06), pages 1174-1185, XP002335022
- ZHU S ET AL.: "Evolutionary origin of inhibitor cystine knot peptides" THE FASEB JOURNAL, [Online] 3 juillet 2003 (2003-07-03), XP002335024 10.1096/fj.02-1044fje Extrait de l'Internet: URL:www.faseb.com> [extrait le 2005-07-06]
- HEITZ A ET AL.: "Solution Structure of the Squash Trypsin Inhibitor MCoTI-II. A New Family for Cyclic Knottins" BIOCHEMISTRY, vol. 40, 2001, pages 7973-7983, XP002335025
- FAJLOUN Z ET AL: "Chemical synthesis and characterization of maurocalcine, a scorpion toxin that activates Ca<2+> release channel/ryanodine receptors" FEBS LETTERS, ELSEVIER SCIENCE PUBLISHERS, AMSTERDAM, NL, vol. 469, no. 2-3, 10 mars 2000 (2000-03-10), pages 179-185, XP004261072 ISSN: 0014-5793
- ESTEVE E ET AL.: "Transduction of the Scorpion Toxin Maurocalcine into Cells" THE JOURNAL OF BIOLOGICAL CHEMISTRY, [Online] vol. 280, no. 13, 14 janvier 2005 (2005-01-14), pages 12833-12839, XP002335026 Extrait de l'Internet: URL:www.jbc.org> [extrait le 2005-07-06]
- MABROUK K ET AL: "Critical amino acid residues of maurocalcine involved in pharmacology, lipid interaction and cell penetration", BIOCHIMICA ET BIOPHYSICA ACTA, vol. 1768, 2007, pages 2528-2540,
- RAM N ET AL: "Design of a gisulfide-less, pharmacologically-inert and chemically-competent analog of maurocalcine for the efficient transport of impermeant compounds into cells", THE JOURNAL OF BIOLOGICAL CHEMISTRY, JBC PAPERS IN PRESS, 21 July 2008 (2008-07-21),

## Description

La présente Invention est relative à l'utilisation de peptides dérivés de la maurocalcine capables de pénétrer dans les cellules comme vecteur pour véhiculer des molécules d'intérêt dans ces cellules.

Le problème du transport de substances, notamment de macromolécules dotées de propriétés pharmacologiques, au travers de la membrane plasmique et de leur accès aux divers compartiments intracellulaires, en particulier le compartiment cytoplasmique et nucléaire, est un obstacle pour la recherche biotechnologique et biomédicale, ainsi que pour l'industrie pharmaceutique.

Parmi les moyens actuellement connus pour introduire des substances dans les cellules, les peptides de translocation également dénommés CPP (*Cell-Penetrating-Peptides*) représentent des vecteurs particulièrement intéressants (Pour une revue voir notamment, Prochiantz, Curr. Opin. Cell. Biol., 2000, 12, 400-406 ; Lindgren et al., Trends Pharmacol. Sci., 2000, 21, 99-102).

En effet, ces molécules de petite taille sont capables de traverser les membranes cellulaires de manière transporteur et récepteur-indépendante et de transporter des macromolécules imperméantes comme les protéines et les acides nucléiques, à faible concentration, sans énergie, de façon efficace (transduction de 100 % des cellules), rapide (de l'ordre de 5 à 15 min), et ce dans tous les types cellulaires, *in vivo* et *in vitro.* En outre, il a été montré que certains de ces peptides étaient capables de franchir la barrière hémato-méningée (Schwarze et Dowdy, Science, 1999, 285, 1569-1572).

Ces vecteurs constitués par un peptide capable de traverser les membranes de manière transporteur et récepteur indépendante sont différents d'autres vecteurs comprenant un glycopeptide ou un peptide couplé au PEG, dans lesquels le peptide chargé positivement sert à condenser l'ADN et le PEG ou le sucre permettent le ciblage des cellules d'intérêt, notamment par liaison du complexe ADN/glycopeptide au récepteur pour le mannose ou l'asialoglycoprotéine (peptides CWCK₁₅CK, CW(CK₃)₄CK et CWK₅CK₅CK₅C (SEQ ID NO : 26 à 28) : Park et al., Bioconjugate Chem., 2002, 13, 232-239 ; Kwok et al., J. Pharm. Sciences, 2003, 92, 11741185). Des peptides de compaction de l'ADN dérivés du peptide CWK₁₈ par substitution d'une à quatre lysines par une cystéine sont également décrits dans McKenzie et al., J. Biol. Chem., 2000, 275, 9970-9977.

Les peptides de pénétration actuellement connus sont regroupés en deux catégories :
* des peptides dérivés de séquences signal de translocation membranaire de différentes protéines (Facteur de croissance fibroblastique dérivé du sarcome de Kaposi (K-FGF) et chaîne légère d'immunoglobuline (Ig(v)) ; le mécanisme de pénétration de ces peptides n'est pas connu, la région hydrophobe centrale est impliquée dans la pénétration mais la structure de cette région varie selon les protéines (hélice alpha (K-FGF) ou feuillet béta (Ig(v)).
* des peptides dérivés de protéines de signalisation intercellulaire ou « protéines messager » ; ces protéines ont la particularité de pénétrer directement dans les cellules et d'atteindre le noyau où elles régulent la transcription (Tat de VIH-1, VP22 de HSV-1 et homéoprotéines).

Des études fonctionnelles ont permis d'identifier des séquences minimales nécessaires et suffisantes pour la translocation de chacun de ces peptides :
- le plus petit fragment des homéoprotéines capable de traverser les membranes et de servir de vecteur à d'autres peptides ou à des oligonucléotides est le peptide 43-58, dénommé pénétratine, correspondant à l'hélice 3 de l'homéodomaine (Derossi et al., J. Biol. Chem., 1994, 269, 10444-10450 et Demande Internationale WO 97/12912). L'étude de mutants de cette séquence a montré que la structure en hélice alpha n'intervient pas dans la translocation intracellulaire mais joue un rôle dans l'adressage nucléaire. En revanche, le résidu W en position 48 est important et les propriétés amphiphiles du peptide sont nécessaires mais pas suffisantes pour la translocation. Des études complémentaires ont confirmé le rôle du résidu W48 et montré l'importance de l'interaction des acides aminés chargés positivement (lysines et arginines) avec les phospholipides membranaires, chargés négativement. Ces études ont conduit à la proposition du modèle du micelle inversé, selon lequel les pénétratines sont stabilisées à la surface de la cellule par des interactions électrostatiques et le tryptophane en position 48 force la formation d'un micelle inversé qui piège le peptide et le délivre dans le cytoplasme.
- le fragment 267-300 de la protéine VP22 correspond à la séquence minimale pour l'internalisation (Elliot et O'Hare, Cell, 1997, 88, 223-233).
- le fragment le plus efficace de la protéine Tat est le fragment 48-60. qui correspond à la totalité de la région basique et inclut le signal de localisation nucléaire. Cependant un fragment plus court (47-57) est capable de transporter, sous forme de protéine de fusion, des protéines de 15 à 120 kDa, dans différents types cellulaires, *in vitro* et *in vivo,* et est capable de franchir la barrière hémato-méningée. En outre, contrairement au peptide Tat du virus humain, le peptide Tat du virus équin possède une structure semblable à celle d'un homéodomaine.

La demande WO 03/106491 décrit des séquences de CPP potentiels identifiés par une méthode de prédiction de séquences de CPP.

Ces études fonctionnelles n'ont pas permis d'identifier un mécanisme général de pénétration de ces peptides, permettant notamment d'identifier les éléments de séquence et/ou de structure communs, responsables de la translocation de ces peptides.

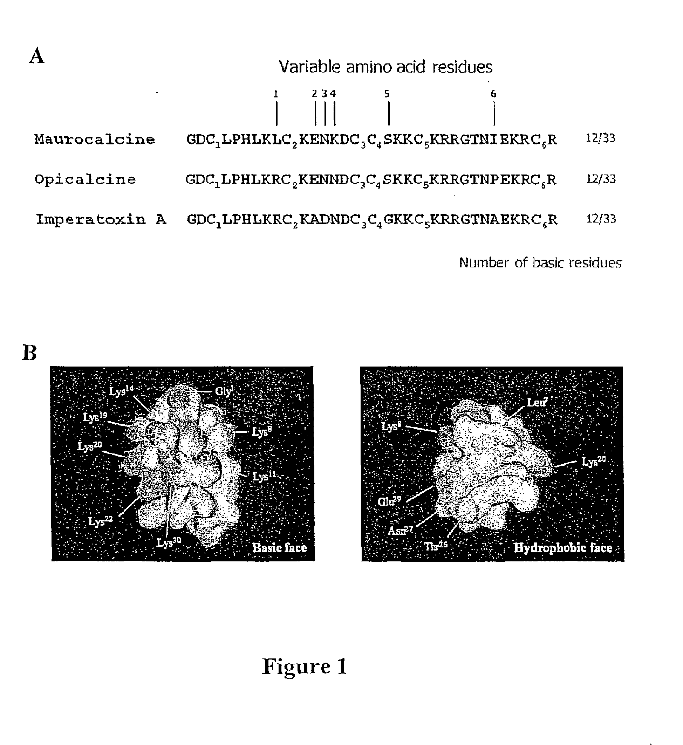

La maurocalcine (MCa) est une toxine de 33 acides aminés, isolée du venin du scorpion *Scorpio maurus palmatus,* correspondant à la séquence SEQ ID NO : 1 dans la liste de séquences jointe en annexe. L'ADNc correspondant code pour un précurseur de 66 acides aminés comprenant 3 domaines : un peptide signal N-terminal de 22 acides aminés, suivi d'un propeptide de 11 acides aminés, riche en acides aminés chargés négativement et terminé par un signal de clivage caractéristique des prohormones (KR), et un peptide C-terminal de 33 acides aminés correspondant au peptide mature (maurocalcine). La maurocalcine présente une forte homologie avec la toxine de deux autres scorpions : l'impératoxine de *Pandinus imperator* (IpTx A, SEQ ID NO : 9 ; 82 % d'identité) et les opicalcines 1 et 2 d'*Opistophtalmus carinatis* (SEQ ID NO : 10 et 11 ; 91 % et 88 % d'identité, respectivement ; figure 1A).

Elle présente également une homologie sur un motif de 6 acides aminés comprenant une succession de résidus basiques, suivi d'une sérine ou d'une thréonine (K₁₉K₂₀-K₂₂R₂₃R₂₄-T₂₆), avec le domaine activateur de la boucle II-III du récepteur de la dihydropyridine (DHPR), un canal calcique voltage-dépendant de type L. Dans le muscle squelettique, le récepteur de la dihydropyridine -situé sur la membrane plasmique- et le récepteur de la ryanodine de type 1 (RyR1) -localisé dans les vésicules du réticulum sarcoplasmique- font partie du complexe de mobilisation du calcium, qui est impliqué dans le couplage excitation-contraction. La maurocalcine est l'un des effecteurs les plus puissants du récepteur de la ryanodine de type 1 (RyR1) ; il a notamment été montré qu'elle stimule la liaison de la ryanodine au récepteur RyR1, qu'elle induit des modifications importantes dans l'ouverture du canal calcique, caractérisées par l'apparition de périodes de sous-conductance prolongées, et que l'addition extracellulaire de maurocalcine à des cultures de myotubes, induit le relargage de calcium du réticulum sarcoplasmique vers le cytoplasme (Fajloun et al., FEBS Letters, 2000, 469, 179-185 ; Estève et al., J. Biol. Chem., 2003, 278, 37822-37831). Ainsi, il a été proposé d'utiliser la maurocalcine ou ses analogues comprenant le motif KKCKKR comme principe actif pour induire une immunosuppression ou traiter des pathologies liées à un dysfonctionnement des canaux calciques (Demande Internationale PCT WO 01/64724).

La structure tridimensionnelle de la maurocalcine correspond à un repliement selon le motif ICK (*Inhibitor Knot Motif*), présent dans de nombreux peptides de plantes, d'animaux ou de champignons ; la famille ICK englobe des peptides de séquences différentes et d'activités biologiques variées, comme des toxines animales (venin de serpent ou d'arachnides) et des inhibiteurs de protéases d'origine végétale comme notamment le peptide McoT I-II (SGSDGGVCPKILKKCRRDSDCPGACICRGNGYCG (SEQ ID NO : 29)), (Zhu et al., The Faseb Journal, 3 juillet 2003 ; Heitz et al., Biochemistry, 2001, 40, 7973-7983).

La structure de la maurocalcine consiste plus précisément en : (i) un noyau compact lié par des ponts disulfures (C₃-C₁₇, C₁₀-C₂₁ et C₁₆-C₃₂) et incluant 3 feuillets béta (9-11, 20-33 et 30-33 ; les feuillets 20-33 et 30-33 étant anti-parallèle), et (ii) une boucle émergeant à l'extrémité N-terminale (Mobash et al., Proteins, 2000, 40, 436-442). Elle est représentée comme une molécule comprenant une face chargée positivement qui pourrait représenter une surface d'interaction avec le récepteur RyR1 (Mobash et al., précité). En outre, l'étude de mutants de la maurocalcine (K8A, K19A, K20A, K22A, R23A, R24A et T26A ; SEQ ID NO : 2 à 8) a montré que le résidu R24 était important pour les effets de la maurocalcine sur la liaison de la ryanodine au récepteur RyR1 (Estève et al., précité).

Au cours de leur étude du rôle effecteur de la maurocalcine sur le récepteur de la ryanodine de type 1 (RyR1), les Inventeurs ont montré que la maurocalcine qui n'appartient à aucune des catégories de protéines contenant des CPP précitées, était capable de pénétrer dans des cellules *in vitro* et de transporter une protéine.

Les Inventeurs ont maintenant cherché à définir les caractéristiques minimales des séquences d'acides aminés dérivées de la maurocalcine capables de servir de vecteur d'intemalisation et d'adressage de substances d'intérêt, notamment des macromolécules d'intérêt comme les protéines et les acides nucléiques et des particules comprenant des molécules chimiques d'intérêt. En outre, la maurocalcine étant une toxine aux propriétés pharmacologiques connues, elle ne peut pas être utilisée *in vivo.* En conséquence, les Inventeurs se sont également donné pour but d'obtenir des vecteurs peptidiques dérivés de la maurocalcine qui, de préférence, ne soient pas toxiques *in vivo,* c'est-à-dire n'ayant pas d'activité pharmacologique sur le récepteur RyR1, notamment du fait qu'ils ne se lient pas audit récepteur RyR1.

La présente invention a en conséquence pour un objet l'utilisation d'un vecteur peptidique pour l'adressage intracellulaire d'une substance d'intérêt, caractérisée en ce que ledit vecteur est essentiellement constitué par un peptide dérivé de la maurocalcine qui est différent de la maurocalcine et qui répond à la séquence (I) suivante :

Z-X₁-X₂-X₃-X₄-X₅-X₆-X₇-X₈-X₉-X₁₀-X₁₁-X₁₂-X₁₃-X₁₄-X₁₅-X₁₆-X₁₇-Z' (I),

dans laquelle :
- X₁ et X₁₇ représentent chacun une cystéine,
- X₄, X₅, X₇ et X₈ représentent chacun une lysine ou une arginine, et
- X₂, X₃, X₆ et X₉ à X₁₆ représentent chacun un acide aminé, pourvu que X₁₅ et/ou X₁₆ représentent une lysine ou une arginine, et
- Z et/ou Z' sont absents ou représentent chacun une séquence de 1 à 35 acides aminés..

L'invention englobe l'utilisation de peptides dérivés de la maurocalcine comme notamment des variants naturels ou synthétiques de la maurocalcine, par exemple des analogues de la maurocalcine. L'invention englobe également l'utilisation de chimères entre la maurocalcine et une toxine comprenant un motif ICK comme par exemple l'opicalcine (1 ou 2) et l'impératoxine A.

Au sens de la présente invention, on entend par acide aminé, un acide aminé naturel ou synthétique, à savoir : les 20 α-acides aminés naturels communément trouvés dans les protéines (A, R, N, D, C, Q, E, G, H, I, L, K, M, F, P, S, T, W, Y et V), certains acides aminés rarement rencontrés dans les protéines (hydroxyproline, hydroxylysine, méthyllysine, diméthyllysine..), les acides aminés qui n'existent pas dans les protéines tels que par exemple la β-alanine, l'acide γ-aminobutyrique, l'homocystéine, l'ornithine, la citrulline, la canavanine, la norleucine, la cyclohexylalanine, ainsi que les énantiomères et les diastéréoisomères des acides aminés précédents.

Le peptide tel que défini dans la présente invention est capable de pénétrer dans n'importe quel type de cellules, *in vitro* ou *in vivo* et de transporter des substances d'intérêt comme des macromolécules imperméantes (protéines, acides nucléiques) et des particules comprenant des molécules chimiques d'intérêt, dans des compartiments cellulaires, plus particulièrement le compartiment cytoplasmique et le compartiment nucléaire. Par exemple, des complexes entre le peptide selon l'invention et des substances telles que des protéines dont le poids moléculaire va jusqu'à au moins 60 kDa et des nanoparticules sont transportées dans le cytoplasme et le noyau des cellules.

Cette propriété peut être aisément vérifiée par incubation dudit peptide lié à la dite substance en présence desdites cellules et détection de la présence dudit peptide et/ou de ladite substance dans les cellules, notamment par analyse d'un marquage spécifique dudit peptide et/ou de ladite substance, par toute technique connue de l'Homme du métier, notamment par microscopie.

En conséquence, ledit peptide est utilisable en tant que vecteur pour l'adressage intracellulaire de substances capables d'interagir avec une cible intracellulaire. Ces substances sont notamment des substances pharmacologiquement actives dont la cible est intracellulaire ; ces drogues sont notamment des médicaments ou des produits phytosanitaires. Ces substances peuvent également être des ligands d'un composant intracellulaire à détecter (molécule endogène ou pathogène), notamment des anticorps ou des fragments d'anticorps (Fab, Fv, scFv), utiles comme sonde moléculaire intracellulaire. Lesdites substances incluent des molécules chimiques, notamment des macromolécules : protéines, peptides, peptides-acides nucléiques (PNA), acides nucléiques (plasmides, oligonucléotides, antisens, siRNA) et des particules, notamment des nanoparticules ou des liposomes comprenant des molécules chimiques d'intérêt, encapsulées ou greffées (couplées) auxdites particules.

Ainsi, ledit peptide a de nombreuses applications dans le domaine des biotechnologies, notamment des nanobiotechnologies, en particulier pour le diagnostic et le traitement de pathologies humaines ou animales (applications biomédicales) et comme outil pour la recherche dans ces domaines.

Pour la mise en oeuvre de la présente invention, la substance à transporter est couplée au vecteur peptidique par tout moyen approprié, connu en lui-même permettant d'associer un peptide à une substance (peptide, protéine, acide nucléique ou autre molécule chimique).

Lorsque ladite substance à transporter est un peptide ou une protéine, elle est avantageusement couplée au vecteur peptidique par une liaison peptidique.

Ladite substance peut également être couplée au vecteur peptidique, de façon non-covalente, notamment par l'intermédiaire de complexes streptavidine-biotine, par exemple le vecteur peptidique est biotinylé et la substance d'intérêt est couplée à la streptavidine.

Ladite substance et ledit vecteur peptidique peuvent également être incorporés à la même particule; ils peuvent notamment être couplés à des nanoparticules ou des liposomes.

En outre, lorsque ledit vecteur est utilisé pour détecter un composant intracellulaire (sonde moléculaire intracellulaire), il peut être avantageusement couplé à un marqueur approprié, à des particules marquées, ou bien à une substance marquée. Le marquage est notamment un marquage fluorescent ou un marquage magnétique, détectable par toute technique connue de l'Homme du métier (microscopie de fluorescence, cytométrie de flux, imagerie de résonance magnétique).

De telles sondes moléculaires intracellulaires ont des applications en imagerie cellulaire *in vitro* et *in vivo,* notamment en temps réel. Elles peuvent notamment être utilisées comme réactif de diagnostic d'une maladie génétique ou acquise ou d'une infection par un microorganisme et comme outil pour la recherche.

Selon un mode de réalisation avantageux de ladite utilisation, X₉ est différent d'une arginine et d'une lysine ; cette mutation permet d'abolir la liaison dudit peptide au récepteur RyR1 et d'abolir les effets pharmacologiques résultant de cette liaison.

Selon un autre mode de réalisation avantageux de ladite utilisation, X₂ et/ou X₆ représentent une cystéine.

Selon une disposition avantageuse de ladite utilisation, X₁ représente une lysine.

Selon un autre mode de réalisation avantageux de ladite utilisation, Z' représente une arginine ou une lysine.

Selon un autre mode de réalisation avantageux de ladite utilisation, Z répond à la séquence (II) suivante :

Z₁-Z₂-Z₃-Z₄-Z₅-Z₆-Z₇-Z₈-Z₉-Z₁₀-Z₁₁-Z₁₂-Z₁₃-Z₁₄-Z₁₅-Z₁₆-Z₁₇-Z₁₈-Z₁₉-Z₂₀-Z₂₁-Z₂₂-Z₂₃-Z₂₄-Z₂₅-Z₂₆-Z₂₇-Z₂₈-Z₂₉-Z₃₀-Z₃₁-Z₃₂-Z₃₃-Z₃₄-Z₃₅ (II),

dans laquelle : Z₁ à Z₃₅ représentent chacun un acide aminé ou sont absents et Z₂₉, Z₃₀, Z₃₂ et Z₃₁ ou Z₃₃ étant toujours présents.

Selon une disposition avantageuse de ce mode de réalisation, Z₂₁ et/ou Z₂₉ représentent une cystéine ; de préférence lorsque Z₂₁ représente une cystéine alors X₂ représente également une cystéine et lorsque Z₂₉ représente une cystéine, alors X₆ représente également une cystéine. De manière préférée X₂, X₆, Z₂₁ et Z₂₉ représentent chacun une cystéine.

Selon une autre disposition avantageuse de ce mode de réalisation, au moins un acide aminé choisi parmi Z₃₀ à Z₃₅ et Z₂₂ à Z₂₈ représente une lysine ou une arginine ; de préférence Z₃₀ et/ou Z₃₄, Z₂₇ et/ou Z₂₈ représentent une arginine ou une lysine.

De manière préférée, au moins trois acides aminés choisis parmi Z₂₂, Z₂₃, Z₂₄, Z₂₅, Z₂₆, Z₂₇, Z₂₈, Z₃₀, Z₃₁, Z₃₂, Z₃₃, Z₃₄ et Z₃₅ représentent chacun une lysine ou une arginine. De manière encore plus préférée, Z₂₇ et Z₃₀ représentent chacun une lysine et Z₂₈ ou Z₃₄ représentent chacun une lysine ou une arginine.

Selon une autre disposition avantageuse de ce mode de réalisation, au moins Z₁ à Z₁₈, Z₂₂, et Z₃₁ ou Z₃₄ sont absents. De préférence, les résidus suivants sont absents : Z₁ à Z₁₈, Z₂₂ et Z₃₁ ou Z₃₄ ; Z₁ à Z₁₉, Z₂₂ et Z₃₁ ou Z₃₄ ; Z₁ à Z₂₀, Z₂₂ et Z₃₁ ou Z₃₄; Z₁ à Z₂₆ et Z₃₁ ou Z₃₄, Z₁ à Z₂₈ et Z₃₁ ou Z₃₄.

Par exemple, l'une des séquences d'acides aminés suivante est présente : Z₁₉ à Z₂₁, Z₂₃ à Z₃₀ et Z₃₂ à Z₃₅; Z₁₉ à Z₂₁, Z₂₃ à Z₃₃ et Z₃₅ ; Z₂₀, Z₂₁, Z₂₃ à Z₃₀ et Z₃₂ à Z₃₅; Z₂₀, Z₂₁ , Z₂₃ à Z₃₃ et Z₃₅ ; Z₂₁, Z₂₃ à Z₃₀ et Z₃₂ à Z₃₅ ; Z₂₁, Z₂₃ à Z₃₃ et Z₃₅.

Selon un autre mode de réalisation avantageux de ladite utilisation, le peptide de séquence (I) est constitué d'acides aminés D.

Des peptides conformes à la présente invention sont représentés notamment par :
a) des chimères entre la maurocalcine et l'impératoxine ou la maurocalcine et l'opicalcine, comme par exemple les peptides de séquence SEQ ID NO : 24 et 25,
b) des peptides de 33 acides aminés dérivés de la maurocalcine dans lesquels :
   - X₁, X₂, X₆, X₁₇, Z₂₁ et Z₂₉ représentent C,
   - X₄, X₅, X₇, X₈, X₁₅, X₁₆, Z₂₇, Z₃₀, et Z₂₈ ou Z₃₄ représentent K ou R ; de préférence au moins X₄, X₅, X₇, X₁₅, Z₂₇ et Z₃₀ représentent K, de manière encore plus préférée X₄, X₅, X₇, X₁₅, Z₂₇ et Z₃₀ représentent K et Z₂₈ ou Z₃₄ représentent K ou R, de préférence Z₂₈ représente R ou Z₃₄ représente K.
   - Z₁ à Z_{18,} Z₂₂, et Z₃₁ ou Z₃₄ sont absents,
   - X₃ représente S ou G
   - X₉ représente R
   - X₁₁ représente T, S ou A
   - X₁₃ représente un acide aminé hydrophobe sélectionné parmi : A, V, L, I, P, W, F et M.
   - X₁₀, X₁₂, X₁₄, Z₁₉, Z₂₀, Z₂₃, Z₂₄, Z₂₅, Z₂₆, Z₃₂, Z₃₃, Z₃₅, Z', et éventuellement Z₂₈, Z₃₁, Z₃₄, représentent A, C, D, E, F, G, K, L, M, P ou N, de préférence A ou F ; par exemple Z₁₉ représente G, et Z' représente R ou K.
c) les fragments des peptides définis en b) correspondant aux positions 16 à 32 ou 16 à 33 de la maurocalcine,
d) les fragments des peptides définis en b) correspondant aux positions 10 à 32 ou 10 à 33 de la maurocalcine,
e) les fragments des peptides définis en b) correspondant aux positions 8 à 32 ou 8 à 33 de la maurocalcine et
f) les peptides dérivés des peptides définis en b), c), d) et e) dans lesquels X₁₅ est différent de R et de K.

Parmi ces peptides on peut citer ceux présentant les séquences SEQ ID NO: 2 et 7 à 23.

Selon un autre mode de réalisation avantageux de ladite utilisation, ledit vecteur peptidique est couplé à un marqueur approprié, notamment un fluorochrome ; le couplage peut être covalent ou non covalent, notamment par l'intermédiaire de complexes streptavidine-biotine marqués ou de particules marquées.

Selon encore un autre mode de réalisation avantageux de ladite utilisation, le vecteur peptidique tel que défini ci-dessus est couplé à des particules, notamment des nanoparticules ; avantageusement lesdites particules sont marquées et/ou elles comprennent une substance d'intérêt, comme une substance pharmacologiquement active, utile notamment comme médicament ou produit phytosanitaire, ou bien une substance ligand d'un composant intracellulaire à détecter, utile comme sonde moléculaire intracellulaire.

Selon un autre mode de réalisation avantageux de ladite utilisation, la séquence (I) du vecteur peptidique tel que défini ci-dessus est fusionnée à une séquence peptidique ou polypeptidique hétérologue d'intérêt, de façon à former un peptide ou une protéine chimérique.

On entend par hétérologue une séquence autre que celle qui est directement adjacente à la séquence (I), dans la séquence de la maurocalcine ou d'un analogue de la maurocalcine.

Ledit peptide ou ladite protéine chimérique sont avantageusement couplés à un marqueur approprié et/ou à des particules, lesdites particules pouvant éventuellement être marquées.

L'insertion de la séquence (I) dans le peptide ou la protéine d'intérêt est effectuée à l'extrémité NH₂ ou COOH ou au niveau d'un site interne approprié, lequel site est choisi en fonction de la structure de ladite protéine ou dudit peptide.

La présente invention a également pour objet une composition comprenant une substance pharmacologiquement active dont la cible est intracellulaire et un vecteur peptidique tel que défini ci-dessus, en tant que vecteur d'adressage intracellulaire de ladite substance.

Selon un mode de réalisation avantageux de ladite composition, ladite substance pharmacologiquement active et ledit vecteur peptidique sont sous la forme d'un peptide ou d'une protéine chimérique tels que définis ci-dessus.

Selon un autre mode de réalisation avantageux de ladite composition, elle comprend des particules comprenant à la fois ladite substance pharmacologiquement active et ledit vecteur peptidique.

Selon encore un autre mode de réalisation avantageux de ladite composition, ladite substance pharmacologiquement active est un médicament destiné à être administré à un individu humain ou animal.

La présente invention a également pour objet la composition telle que définie ci-dessus comme médicament.

La présente invention a également pour objet l'utilisation d'une composition telle que définie ci-dessus, pour la préparation d'un médicament, destiné au traitement d'une pathologie chez l'homme ou l'animal.

La présente invention a également pour objet une composition comprenant un ligand d'un composant intracellulaire à détecter (sonde intracellulaire) et un vecteur peptidique tel que défini ci-dessus, en tant que vecteur d'adressage intracellulaire dudit ligand.

Selon un mode de réalisation avantageux de ladite composition, ledit vecteur peptidique est couplé à un marqueur approprié, à des nanoparticules marquées, et/ou au dit ligand (protéine ou peptide chimérique), comme précisé ci-dessus.

Selon un autre mode de réalisation avantageux de ladite composition, ledit ligand est un anticorps ou un fragment fonctionnel d'anticorps dirigé contre ledit composant.

La présente invention a également pour objet la composition telle que définie ci-dessus comme réactif de diagnostic.

La présente invention a également pour objet une méthode *in vitro* de détection d'un composant intracellulaire, caractérisée en ce qu'elle comprend :
- la mise en contact d'un échantillon cellulaire avec un réactif de détection tel que défini ci-dessus, et
- la détection d'un marquage intracellulaire, par tout moyen approprié.

Conformément à l'invention, ledit échantillon cellulaire comprend des cellules d'un organisme eucaryote supérieur, éventuellement infectées par un microorganisme, ou bien des cellules d'un microorganisme (bactérie, levure, champignon, parasite).

Selon un mode de mise en oeuvre avantageux de ladite méthode *in vitro,* ledit réactif de détection est un réactif marqué tel que défini ci-dessus. Le dit réactif est par exemple couplé à un fluorochrome ou à des particules couplées à un fluorochrome.

Conformément à l'invention, ledit organisme est eucaryote supérieur, notamment un être humain, un animal ou une plante.

La présente invention a également pour objet, un peptide de séquence (I) telle que définie ci-dessus, à l'exception des peptides SEQ ID NO : 1 à 11, 26 et 27 et STSRKLKS(PO₃H₂)QGTRRGKNRTPHKGVKRGCSKRKYRKSSLKSRKRCDDANRNFRSHL ; ledit peptide peut avantageusement être marqué.

La présente invention a également pour objet des particules couplées à un vecteur peptidique tel que défini ci-dessus ; lesdites particules et/ou ledit vecteur peuvent avantageusement être marquées. Avantageusement, lesdites particules sont des nanoparticules.

La présente invention a également pour objet un polynucléotide codant pour le vecteur peptidique, ou bien le peptide tel que défini ci-dessus. Conformément à l'invention, la séquence dudit polynucléotide (ADN ou ARN) correspond à celle de l'ADNc codant pour ledit vecteur peptidique ou bien pour ledit peptide. La séquence dudit polynucléotide peut comprendre un peptide signal de translocation dans le réticulum endoplasmique, de façon à produire un vecteur peptidique/peptide sécrétés dans le milieu extracellulaire. Ledit peptide signal peut notamment être celui de la maurocalcine ou de n'importe quel peptide ou protéine capable d'être sécrété dans le milieu extracellulaire. En outre, la séquence dudit polynucléotide peut également comprendre la séquence de 11 acides aminés du propeptide de la maurocalcine ou d'un autre peptide de la famille ICK, insérée en 5' de la séquence d'ADNc codant pour ledit vecteur peptidique.

Ladite séquence peut avantageusement être modifiée de façon à ce que l'usage des codons soit optimal chez l'hôte dans lequel elle est exprimée.

L'objet de la présente invention englobe en particulier :
a) des cassettes d'expression comprenant au moins un polynucléotide tel que défini ci-dessus, sous le contrôle de séquences régulatrices de la transcription et éventuellement de la traduction appropriées (promoteur, activateur, intron, codon d'initiation (ATG), codon stop, signal de polyadénylation), et
b) des vecteurs recombinants comprenant un polynucléotide conforme à l'invention. Avantageusement ces vecteurs sont des vecteurs d'expression comprenant au moins une cassette d'expression telle que définie ci-dessus.

La présente invention a en outre pour objet, des cellules hôtes procaryotes ou eucaryotes modifiées par au moins un polynucléotide ou un vecteur recombinant tel que défini ci-dessus.

La présente invention a en outre pour objet, un mammifère non-humain transgénique, caractérisé en ce que tout ou partie de ses cellules sont modifiées par un polynucléotide ou un vecteur recombinant tel que défini ci-dessus.

La présente invention a également pour objet, une plante transgénique, caractérisée en ce que tout ou partie de ses cellules sont modifiées par un polynucléotide ou un vecteur recombinant tel que défini ci-dessus.

De nombreux vecteurs d'acides nucléiques dans lesquels on peut insérer une molécule d'acide nucléique d'intérêt afin de l'introduire et de la maintenir dans une cellule hôte eucaryote ou procaryote, sont connus en eux-mêmes ; le choix d'un vecteur approprié dépend de l'utilisation envisagée pour ce vecteur (par exemple réplication de la séquence d'intérêt, expression de cette séquence, maintien de cette séquence sous forme extrachromosomique, ou bien intégration dans le matériel chromosomique de l'hôte), ainsi que de la nature de la cellule hôte. Par exemple, on peut utiliser entre autres des vecteurs viraux tels que les adénovirus, les rétrovirus, les lentivirus, les AAV et les baculovirus, dans lesquels a été insérée, préalablement, la séquence d'intérêt ; on peut également associer ladite séquence (isolée ou insérée dans un vecteur plasmidique) avec une substance lui permettant de franchir la membrane des cellules hôtes, telle qu'un transporteur comme un nanotransporteur ou une préparation de liposomes, ou de polymères cationiques, ou bien l'introduire dans ladite cellule hôte en utilisant des méthodes physiques telles que l'électroporation ou la microinjection. En outre, on peut avantageusement combiner ces méthodes, par exemple en utilisant l'électroporation associée à des liposomes.

Les polynucléotides, les vecteurs recombinants et les cellules transformées tels que définis ci-dessus, sont utiles notamment pour la production des peptides et protéines de fusion selon l'invention.

Les polynucléotides selon l'invention sont obtenus par les méthodes classiques, connues en elles-mêmes, en suivant les protocoles standards tels que ceux décrits dans *Current Protocols in Molecular Biology* (*Frederick M. AUSUBEL, 2000*, *Wiley and son Inc, Library of Congress, USA*). Par exemple, ils peuvent être obtenus par amplification d'une séquence nucléique par PCR ou RT-PCR, par criblage de banques d'ADN génomique par hybridation avec une sonde homologue, ou bien par synthèse chimique totale ou partielle. Les vecteurs recombinants sont construits et introduits dans des cellules hôtes par les méthodes classiques d'ADN recombinant et de génie génétique, qui sont connues en elles-mêmes.

Les peptides et leurs dérivés (peptides et protéines de fusion) sont préparés par les techniques classiques connues de l'Homme du métier :
- les peptides dérivés de la maurocalcine et les peptides de fusion peuvent être synthétisés en phase solide, selon la technique Fmoc, originellement décrite par Merrifield et al. (J. Am. Chem. Soc., 1964, 85: 2149-) et purifiés par chromatographie liquide haute performance en phase inverse,
- les peptides dérivés de la maurocalcine, ainsi que les peptides et les protéines de fusion peuvent également être produits à partir des ADNc correspondants, obtenus par tout moyen connu de l'homme du métier ; l'ADNc est cloné dans un vecteur d'expression eucaryote ou procaryote et la protéine ou le fragment produits dans les cellules hôte modifiées par le vecteur recombinant sont purifiés par tout moyen approprié, notamment par chromatographie d'affinité.

Outre les dispositions qui précèdent, l'invention comprend encore d'autres dispositions qui ressortiront de la description qui va suivre, qui se réfère à des exemples de mise en oeuvre des peptides selon la présente invention ainsi qu'au Tableau ci-après résumant les séquences de la Demande et aux dessins annexés dans lesquels :
- la figure 1 représente la séquence et la structure de la maurocalcine. A. Alignement des séquences de la maurocalcine et de deux autres toxines analogues, également actives sur le récepteur de la ryanodine : l'opicalcine 1 et l'impératoxine A. Toutes les toxines possèdent le même nombre d'acides aminés chargés positivement ou acides aminés basiques (12 acides aminés chargés positivement sur un total de 33 acides aminés). B. Structure tridimensionnelle de la maurocalcine, établie à l'aide du logiciel WebLab ViewerPro™. Panneau de gauche : face basique incluant les résidus chargés positivement (G₁, K₈, K₁₁, K₁₄, K₁₉, K₂₀, K₂₂ et K₃₀). Panneau de droite : face hydrophobe qui illustre l'absence de résidus chargés positivement sur la face opposée de la molécule. Le squelette peptidique est représenté par un ruban, alors que les chaînes latérales des résidus chargés positivement sont indiqués par des boules et des tiges représentés à l'échelle.

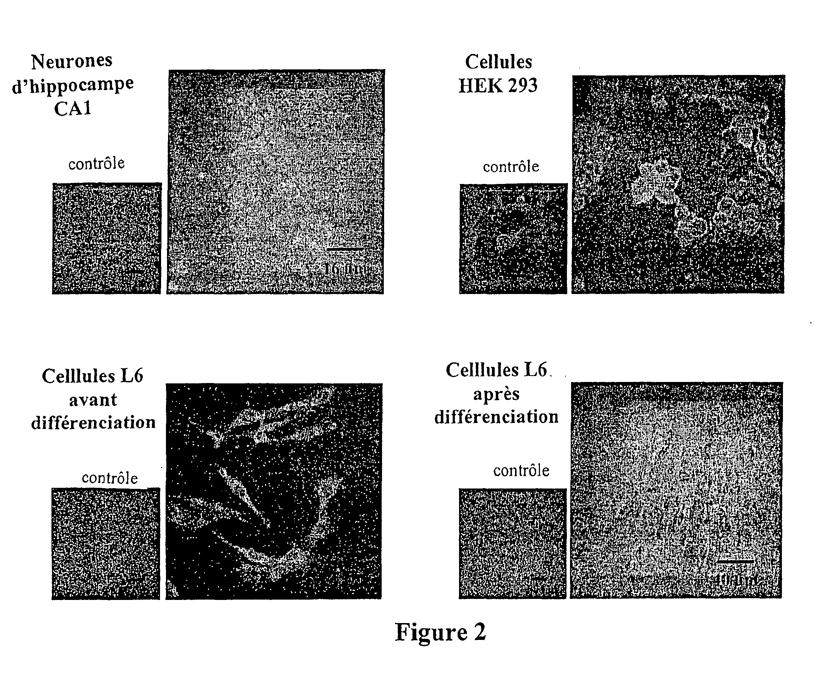

- la figure 2 montre que le complexe MCa_{b}/Strept-Cy3 transduit de façon efficace de nombreuses cultures de cellules différentes. Des cultures de neurones d'hippocampe CA1, de cellules HEK293 et de cellules L6 non-différenciées ont été incubées pendant 30 min avec 100 nM de maurocalcine biotinylée, complexée à de la streptavidine marquée à la cyanine 3 (MCa_{b}/Strept-Cy3), puis les cellules ont été fixées et analysées en microscopie confocale. Des cellules L6 différenciées ont été traitées de façon similaire. Les panneaux quatre fois plus petits représentent les expériences contrôles, dans lesquelles les cellules ont été traitées avec un mélange de MCa non biotinylée et de Strept-Cy3.

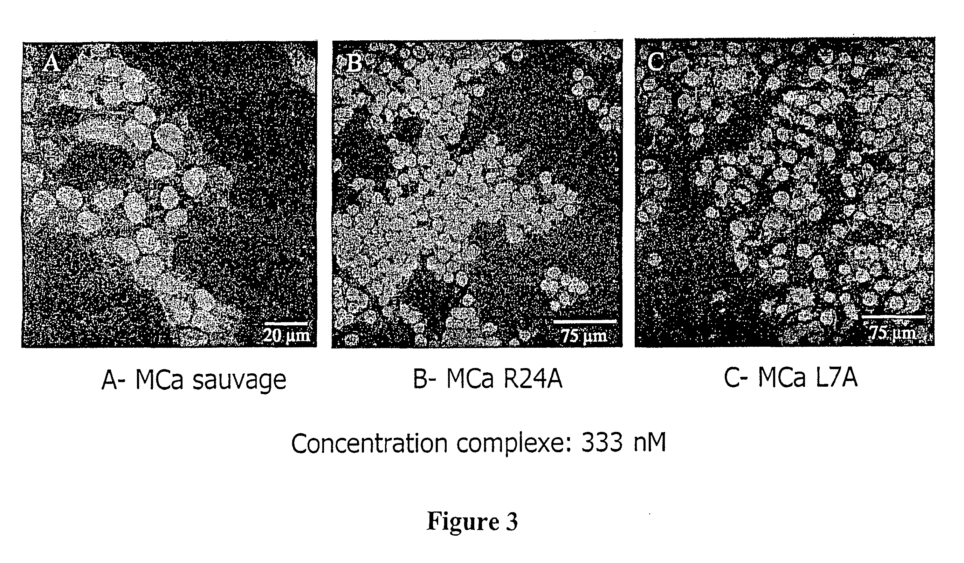

- la figure 3 montre que les complexes entre les variants R24A et L7A de la maurocalcine biotinylés et la streptavidine marquée à la cyanine transduisent de façon efficace des cultures de cellules. Des cultures de cellules HEK293 ont été incubées pendant 30 min avec 333 nM de variant R24A, L7A ou de maurocalcine, biotinylés et complexés à de la streptavidine marquée à la cyanine 3 (MCa_{b}/Strept-Cy3), puis les cellules ont été fixées et analysées en microscopie confocale.

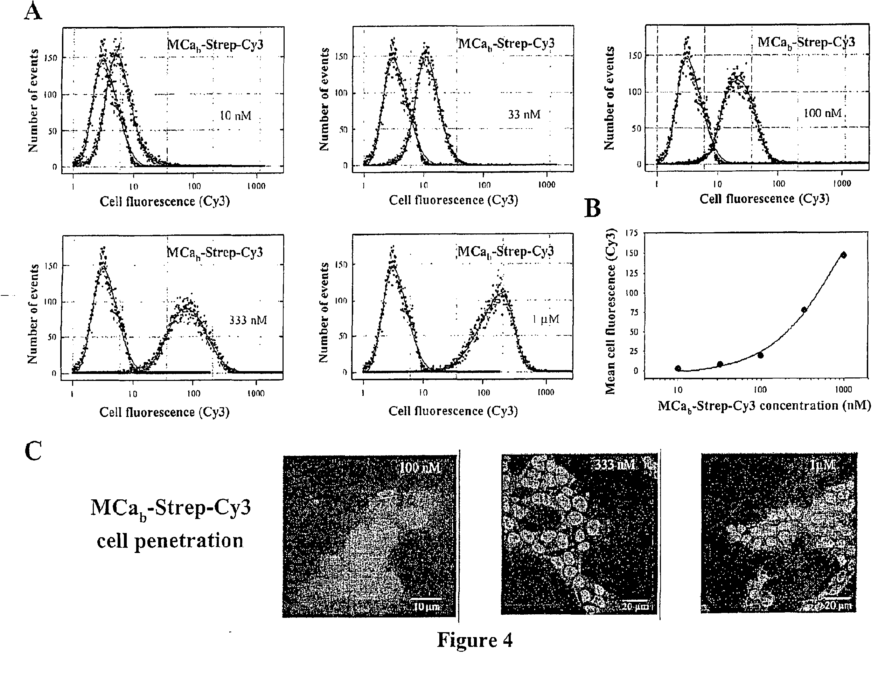

- la figure 4 montre que la pénétration des complexes MCa_{b}/Strept-Cy3 est dose-dépendante. A. Analyse en cytométrie de flux de la pénétration des complexes MCa_{b}/Strept-Cy3 aux concentrations indiquées (10 nM, 33 nM, 100 nM, 333 nM et 1 µM). Les cellules ont été traitées à la trypsine (1 mg/ml), avant l'analyse en cytométrie de flux. B. Fluorescence cellulaire moyenne, en fonction de la concentration de complexes MCa_{b}/Strept-Cy3. Les valeurs correspondent à l'équation suivante y = y₀+aX(1-exp(-bXx)) dans laquelle y₀ = -3,8, a = 199 et b = 1,5X10⁻³. C. Images d'immunofluorescence confocale de cellules HEK293 incubées pendant 1 heure avec différentes concentrations de complexes MCab-Strep-Cy3. Les noyaux sont colorés par le To-PRO-3.

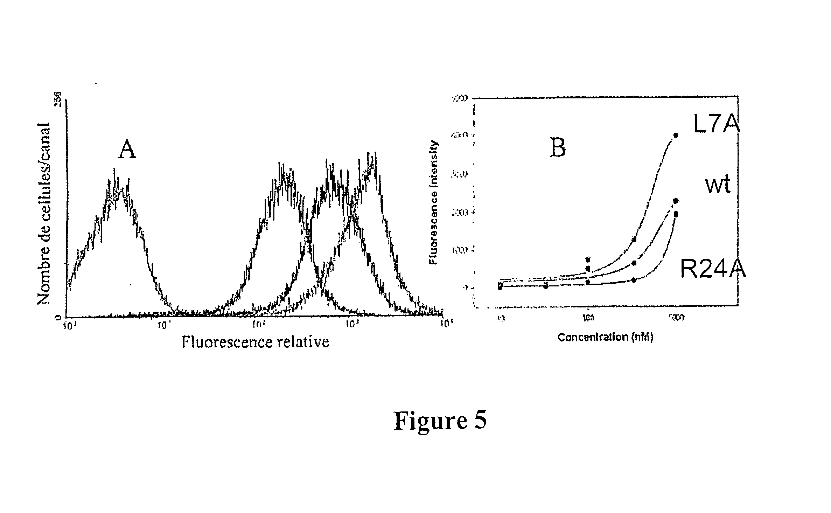

- la figure 5 montre que la pénétration des complexes entre les variants de la maurocalcine biotinylés et la streptavidine marquée à la cyanine est dose-dépendante. A. Analyse en cytométrie de flux de la pénétration des complexes entre les variants R24A, L7A ou la maurocalcine, biotinylés et la Strept-Cy3 Les cellules ont été traitées à la trypsine (1 mg/ml), avant l'analyse en cytométrie de flux. B. Fluorescence cellulaire moyenne, en fonction de la concentration de complexes.

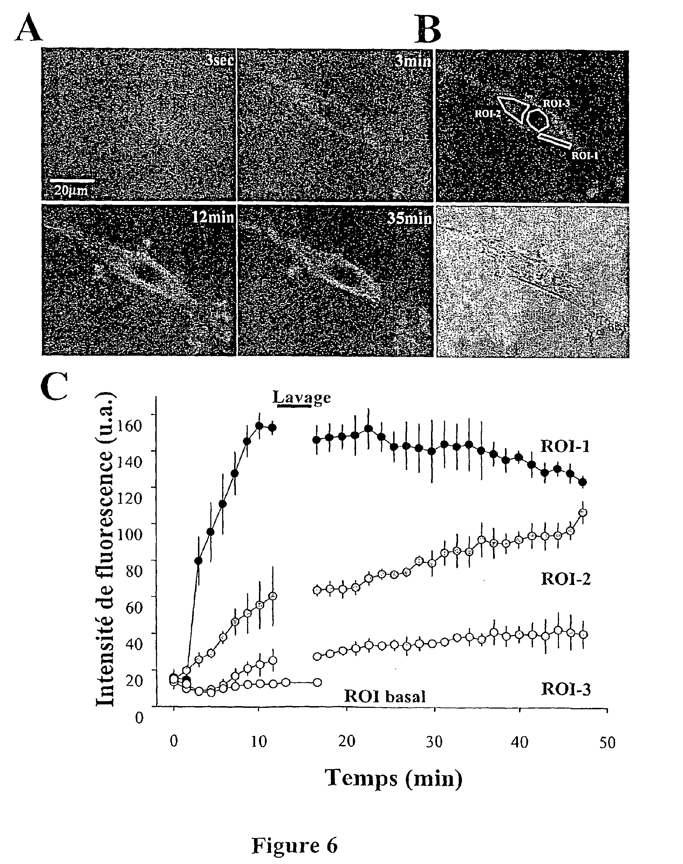

- la figure 6 représente la cinétique de transduction du complexe MCa_{b}/Strept-Cy3 dans des cellules L6 non-différenciées, sur une durée d'une heure. (A) Image confocale de la fluorescence Cy3 d'une cellule L6 non-différenciée, 3 sec, 3 min et 12 min après l'addition de 100 nM de MCa_{b}/Strept-Cy3 et 23 min après lavage du complexe (35 min au total). (B) Position des différentes régions d'intérêt (ROI) sur l'image de la lumière transmise par la cellule (contraste d'interférence différentiel, panneau supérieur) et position correspondante de l'image confocale de la fluorescence Cy3. (C) Evolution de l'intensité de la fluorescence Cy3 normalisée à l'unité de surface des régions d'intérêt, en fonction du temps, dans les différents compartiments des cellules L6. Le complexe MCa_{b}/Strept-Cy3 a été appliqué à la concentration de 100 nM dans le milieu à t=0, puis à t=12 min, il a été éliminé du milieu par des lavages pendant 5 min. Le « ROI basal » correspond à l'intensité de fluorescence du milieu extracellulaire. Les valeurs représentent les moyennes ± écart type de 5 cellules différentes. Des résultats similaires ont été observés dans les trois différentes préparations de cellules.

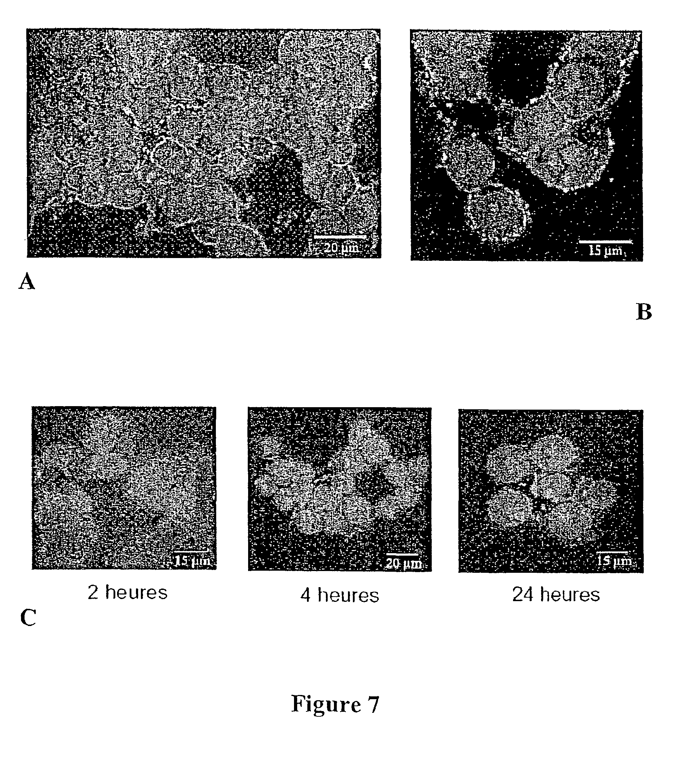

- la figure 7 illustre la localisation subcellulaire des complexes MCa_{b}-Strep-Cy5, analysée par immunofluorescence en microscopie confocale et comparée à la localisation de la concanavaline A(A), la tubuline alpha (B), et en fonction du temps (C). A. Localisation sub-cellulaire des complexes MCa_{b}-Strep-Cy5 dans les cellules HEK293, par comparaison avec un marqueur de la membrane plasmique. Les cellules ont été incubées pendant une heure en présence de complexes MCa_{b}-Strep-Cy5 (333 nM). La membrane plasmique est marquée par la concanvaline et les noyaux avec l'iodure de propidium. Les images représentent un seul plan confocal. B. Comme en (A) mais par comparaison avec un marqueur du cytosquelette (anticorps anti-tubuline alpha). C. Modifications de la distribution cellulaire des complexes MCa_{b}-Strep-Cy5 après la translocation cellulaire. Images de fluorescence confocale de Cy3 (complexes MCa_{b}-Strep-Cy3) et To-PRO (noyaux) de cellules HEK293 incubées avec 333 nM de complexes, pendant 2 heures, 4 heures et 24 heures. Un marquage progressif des noyaux par les complexes MCa_{b}-Strep-Cy5 est observé.

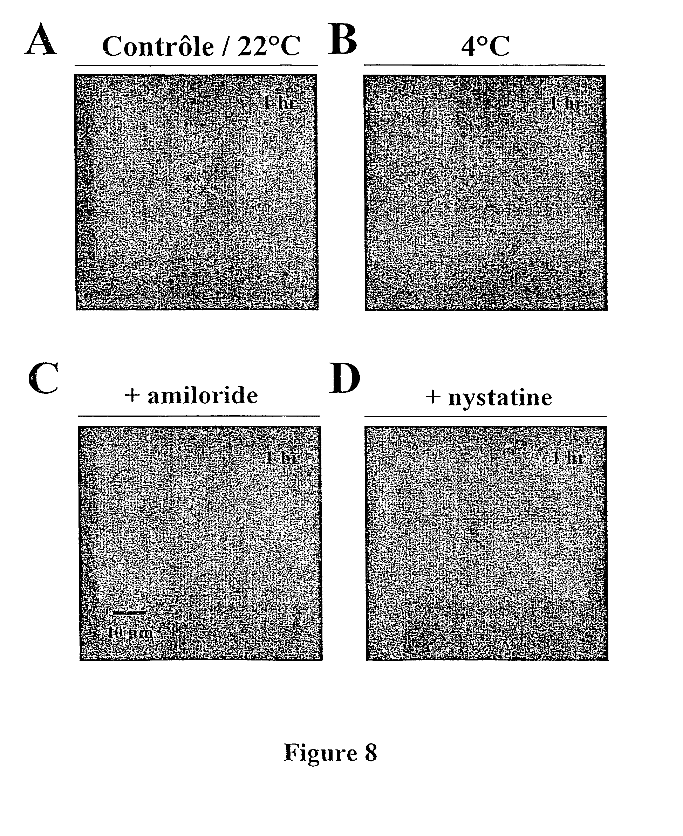

- la figure 8 montre que l'entrée dans la cellule du complexe MCa_{b}/Strept-Cy3 ne nécessite pas d'énergie. (A) Condition contrôle pour l'entrée du complexe MCa_{b}/Strept-Cy3 dans les cellules HEK293. Les cellules ont été incubées en présence de 100 nM de complexe, pendant 1h à 22°C. Les cellules ont été fixées et les images ont été acquises par microscopie confocale. (B) Effet de la température sur la pénétration du complexe MCa_{b}/Strept-Cy3. Les cellules ont été incubées et observées dans les mêmes conditions expérimentales qu'à 22°C. (C) Effet de la nystatine (50 µM) sur la pénétration du complexe MCa_{b}/Strept-Cy3 dans les cellules HEK293. La nystatine est un inhibiteur de la pinocytose. Les conditions sont identiques à celles utilisées en (A). (D) Effet de l'amiloride (3 mM) sur la pénétration du complexe MCa_{b}/Strept-Cy3 dans les cellules HEK293. L'amiloride est un inhibiteur de l'endocytose des cavéoles. Les conditions sont identiques à celles utilisées en (A).

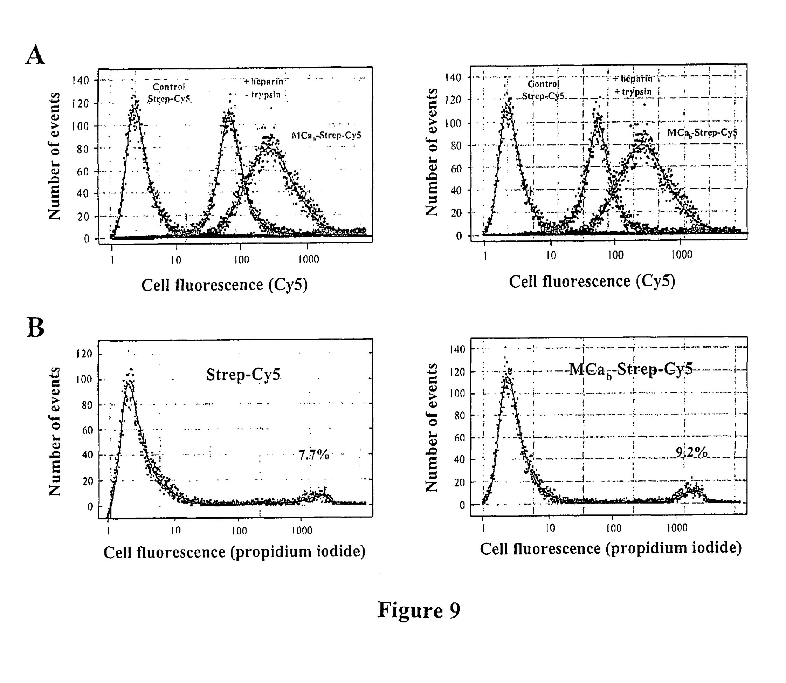

- la figure 9 illustre l'analyse en cytométrie de flux de l'effet du traitement à l'héparine et/ou à la trypsine sur la pénétration cellulaire des complexes MCa_{b}-Strep-Cy5 (A) et la toxicité cellulaire des complexes (B). (A) L'incubation des cellules et des complexes MCa_{b}-Strep-Cy5 avec 10 µg/ml d'héparine réduit la pénétration du complexe (panneau de gauche). La valeur de fluorescence moyenne de Cy5 est de 307 sans traitement, alors qu'en présence d'héparine, elle descend à 78. Le traitement des cellules et des complexes MCa_{b}-Strep-Cy5 avec 10 µg/ml d'héparine, combiné avec le traitement des cellules par la trypsine (1mg/ml) diminue encore la fluorescence moyenne jusqu'à la valeur de 64. La fluorescence contrôle, après traitement avec Strp-Cy5 seule atteint la valeur de 3,1 et n'est pas influencée par le traitement à la trypsine ou à l'héparine. La pénétration des complexes MCa_{b}-Strep-Cy5 est testée à la concentration de 1 µM. (B) La toxicité cellulaire de 1 µM de complexe Strep-Cy5 ou MCa_{b}-Strep-Cy5, analysée par l'incorporation cellulaire de l'iodure de propidium. La proportion de cellules marquées à l'iodure de propidium est indiquée en pourcentages.

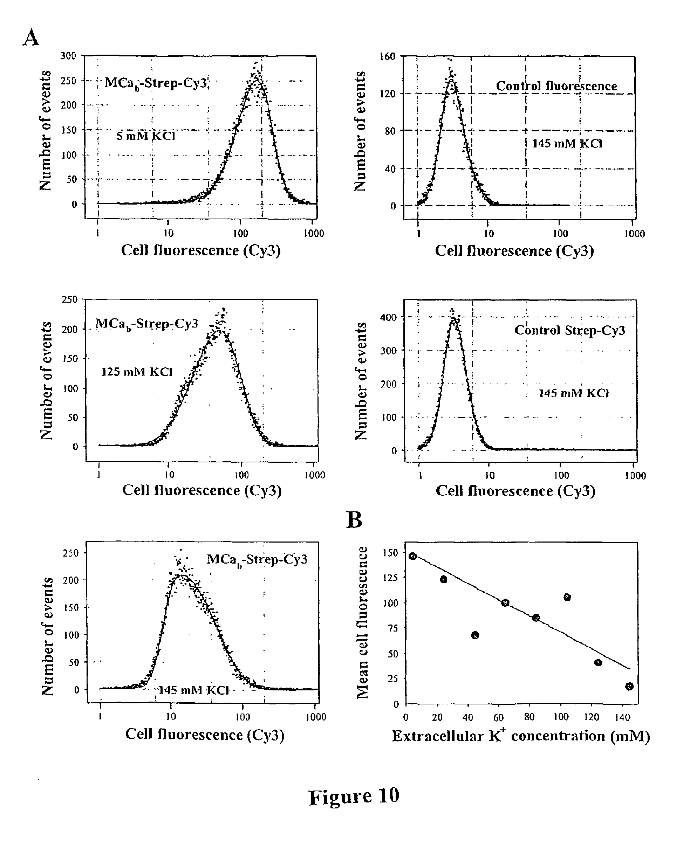

- la figure 10 illustre l'effet de l'augmentation de la concentration extracellulaire en K+ sur la pénétration cellulaire des complexes MCa_{b}-Strep-Cy3 (A,B). (A) Analyse en cytométrie de flux des effets de l'augmentation des concentrations en KCl sur la pénétration cellulaire des complexes MCa_{b}-Strep-Cy3. Les panneaux de droite correspondent aux contrôles (fluorescence cellulaire sans complexe (en haut) et avec Strep-Cy3 (en bas)), en présence de 145 mM KCI. Le gradient de KCl n'a pas d'effet sur les valeurs des contrôles. Les panneaux de gauche illustrent l'effet de 5 mM (en haut, valeur de fluorescence moyenne de 145), 125 mM (milieu, valeur de fluorescence moyenne de 40) et 145 mM KCl (en bas, valeur de fluorescence moyenne de 17), respectivement. (B) Evolution de la fluorescence moyenne en fonction de la concentration en KCl. Régression linéaire des valeurs avec y=y₀ + aX x avec une fluorescence maximale y₀= 139,5 et une pente décroissante a= -0,72.

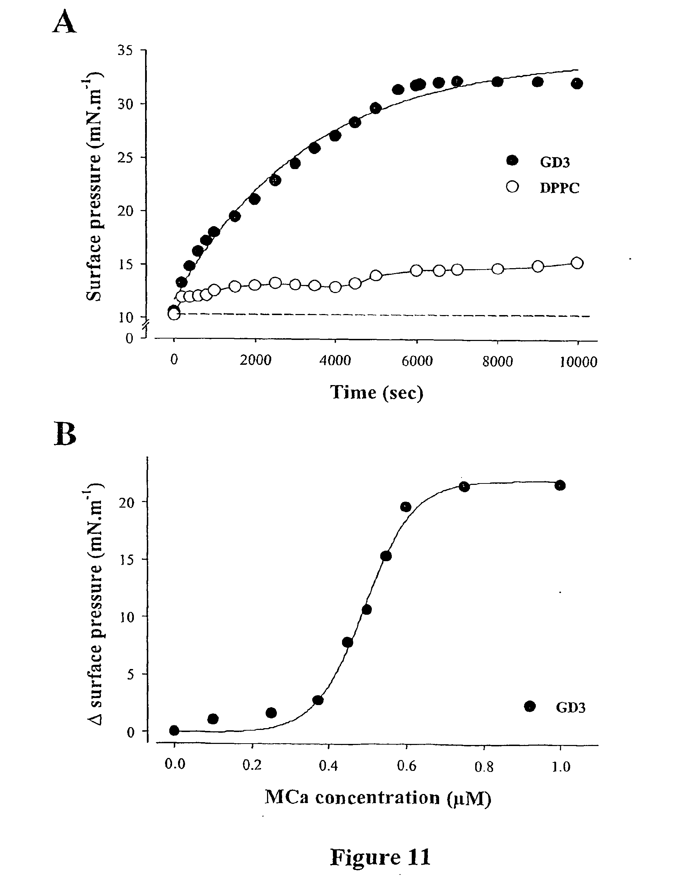

- la figure 11 représente l'interaction de MCa avec les lipides membranaires (A,B). A. Mesure de la pression de surface de films monomoléculaires de GD3 et de DPPC. Cinétiques des changements de la pression de surface, induits par l'application de 1 µM MCa. Les résultats illustrent l'interaction de MCa avec GD3 mais pas DPPC. La pression de surface initiale était de l'ordre de 10 mN.m⁻¹. Les résultats correspondent à une augmentation sigmoïdale vers un maximum, selon l'équation y=y₀ + a X (1 -exp(-b X x)) dans laquelle la pression de surface initiale y₀= 11,6 mN.m⁻¹, l'augmentation maximale de pression de surface a= 22,9 mN.m⁻¹ et la constante de temps du changement de la pression de surface b + 3 X 10-4 sec⁻¹. (B) Evolution de la pression de surface des films de GD3 en fonction de la concentration en MCa. Les résultats correspondent à une fonction sigmoïde y= a/(1+ exp(-(x-x₀)/b)) dans laquelle l'augmentation maximale de la pression de surface a= 22,1 mN.m⁻¹, la pente b=0,006 et 50 % de l'effet maximum est obtenu à la concentration X₀= 0,49 µM

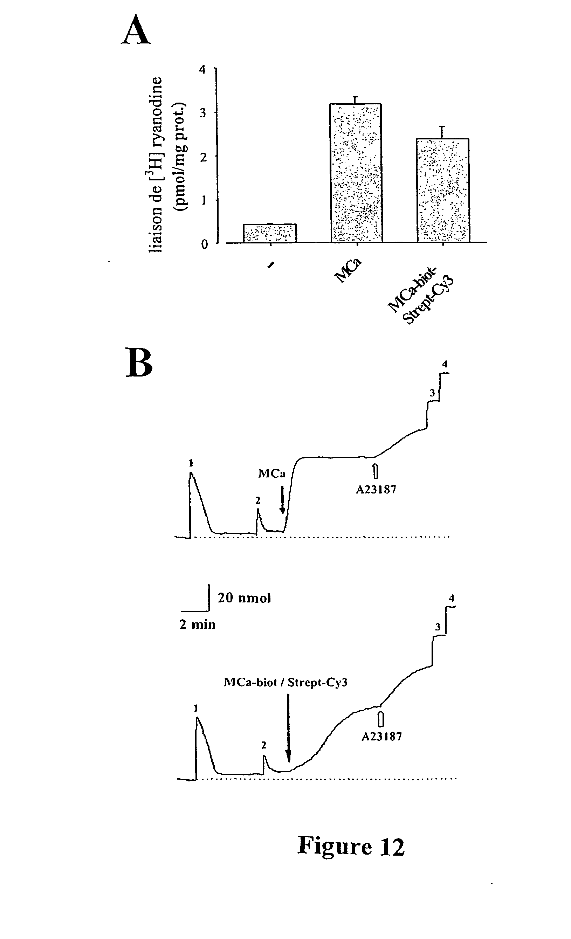

- la figure 12 illustre l'effet du complexe MCa_{b}/Strept-Cy3 sur le récepteur RyR1. (A) Stimulation de la liaison de la [³H]-ryanodine par 100 nM de complexe MCa_{b}/Strept-Cy3. La liaison spécifique de la ryanodine tritiée sur les vésicules du réticulum sarcoplasmique a été mesurée comme décrit dans le matériel et méthodes. (-) représente la liaison en absence de MCa ou de complexe MCa_{b}/Strept-Cy3. (B) Induction du relargage de calcium par les vésicules du réticulum sarcoplasmique par 100 nM de complexe MCa_{b}/Strept-Cy3. 1 et 2 représentent l'addition de respectivement, 50 et 20 µM de CaCl₂ (concentration finale), alors que 3 et 4 représentent l'addition consécutive de 20 µM de CaCl₂. A23187 a été ajouté à une concentration finale de 4 µM.

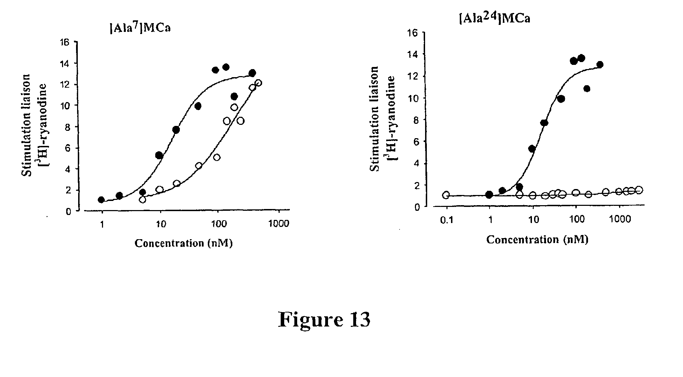

- la figure 13 illustre l'effet des variants L7A et R24A de la maurocalcine, biotinylés et complexés à Strep-Cy3 sur le récepteur RyR1. La stimulation de la liaison de la [³H]-ryanodine a été mesurée à des concentrations croissantes de complexes. La liaison spécifique de la ryanodine tritiée sur les vésicules du réticulum sarcoplasmique a été mesurée comme décrit dans le matériel et méthodes.

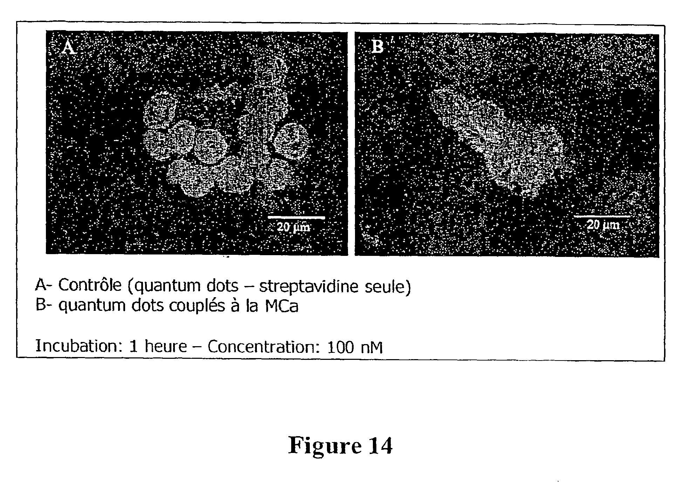

- la figure 14 illustre l'utilisation de la maurocalcine pour la pénétration cellulaire de nanoparticules. A. Des cultures de cellules HEK293 ont été incubées pendant 1 heure avec 100 nM de maurocalcine couplée à des nanoparticules (Qdot^{®}, QUANTUMDOT CORPORATION), puis les cellules ont été fixées et analysées en microscopie confocale. Des cultures de cellules traitées dans les mêmes conditions avec des Qdots couplés à la streptavidine servent de contrôle.

**Tableau I : Liste des séquences**

| Numéro d'identification | numéro d'accès NCBI | peptide | séquence |
|---|---|---|---|
| SEQ ID NO : 1 | 1C6WA | Maurocalcine¹ (MCa) | GDCLPHLKLCKENKDCCSKKCKRRGTNIEKRCR |
| SEQ ID NO : 2 | - | Mutant MCa K8A | GDCLPHLALCKENKDCCSKKCKRRGTNIEKRCR |
| SEO ID NO : 3 | - | Mutant MCa K19A | GDCLPHLKLCKENKDCCSAKCKRRGTNIEKRCR |
| SEQ ID NO : 4 | - | Mutant MCa K20A | GDCLPHLKLCKENKDCCSKACKRRGTNIEKRCR |
| SEQ ID NO: 5 | - | Mutant MCa K22A | GDCLPHLKLCKENKDCCSKKCARRGTNIEKRCR |
| SEQ ID NO : 6 | - | Mutant MCa R23A | GDCLPHLKLCKENKDCCSKKCKARGTNIEKRCR |
| SEQ ID NO : 7 | - | Mutant MCa R24A | GDCLPHLKLCKENKDCCSKKCKRAGTNIEKRCR |
| SEQ ID NO : 8 | - | Mutant MCa T26A | GDCLPHLKLCKENKDCCSKKCKRRGANIEKRCR |
| SEQ ID NO : 9 | P59868 | Impératoxine A ² (IpTxA) | GDCLPHLKRCKADNDCCGKKCKRRGTNAEKRCR |
| SEQ ID NO : 10 | P60252 | Opicalcine 1 (Opi) | GDCLPHLKRCKENNDCCSKKCKRRGTNPEKRCR |
| SEQ ID NO : 11 | P60253 | Opicalcine 2 (Opi) | GDCLPHLKRCKENNDCCSKKCKRRGANPEKRCR |
| SEQ ID NO :12 | - | Mutant MCa D2A | GACLPHLKLCKENKDCCSKKCKRRGTNIEKRCR |
| SEQ ID NO :13 | - | Mutant MCa L4A | GDCAPHLKLCKENKDCCSKKCKRRGTNIEKRCR |
| SEQ ID NO: 14 | - | Mutant MCa P5A | GDCLAHLKLCKENKDCCSKKCKRRGTNIEKRCR |
| SEQ ID NO : 15 | - | Mutant MCa H6A | GDCLPALKLCKENKDCCSKKCKRRGTNIEKRCR |
| SEQ ID NO : 16 | - | Mutant MCa L7A | GDCLPHAKLCKENKDCCSKKCKRRGTNIEKRCR |
| SEQ ID NO :17 | - | Mutant MCa L9A | GDCLPHLKACKENKDCCSKKCKRRGTNIEKRCR |
| SEQ ID NO : 18 | - | Mutant MCa E12A | GDCLPHLKLCKANKDCCSKKCKRRGTNIEKRCR |
| SEQ ID NO :19 | - | Mutant MCa N13A | GDCLPHLKLCKEAKDCCSKKCKRRGTNIEKRCR |
| SEQ ID NO : 20 | - | Mutant MCa D15A | GDCLPHLKLCKENKACCSKKCKRRGTNIEKRCR |
| SEQ ID NO : 21 | - | Mutant MCa G25A | GDCLPHLKLCKENKDCCSKKCKRRATNIEKRCR |
| SEQ ID NO : 22 | - | Mutant MCa N27A | GDCLPHLKLCKENKDCCSKKCKRRGTAIEKRCR |
| SEQ ID NO : 23 | - | Mutant MCa E29A | LPHLKLCKENKDCCSKKCKRRGTNIAKRCR |
| SEQ ID NO : 24 | - | Chimère Mca/lpTxA | GDCLPHLKRCKADNDCCSKKCKRAGTNIEKRCR |
| SEQ ID NO : 25 | - | Chimère Mca/lOpi | GDCLPHLKRCKENNDCCSKKCKRAGTNIEKRCR |
| SEQ ID NO : 26 | - | Peptide de compaction de l'ADN | CWCK₁₅CK |
| SEQ ID NO : 27 | - | Peptide de compaction de l'ADN | CW(CK₃)₄CK |
| SEQ ID NO: 28 | - | Peptide de compaction de l'ADN | CWK₅CK₅CK₅C |
| SEQ ID NO : 29 | - | Peptide ICK de plantes | SGSDGGVCPKILKKCRRDSDCPGACICRGNGYCG |

| | | | |
|---|---|---|---|
| 1 : toxine de *Scorpio maurus palmatus*; 2: toxine de *Pandinus imperator;* 3 et 4: toxine d'*Opistophthalmus carinatus* | | | |

### EXEMPLE 1 : Analyse de la pénétration dans les cellules de complexes entre la streptavidine marquée et la maurocalcine ou un peptide dérivé selon l'invention, biotinylés.

### 1) Matériels et méthodes

### a) Synthèse peptidique

La maurocalcine, les peptides issus de la maurocalcine selon la présente invention et leurs dérivés biotinylés ont été préparés par synthèse peptidique en phase solide (Merrifield, Science, 1986, **232**, 341-347), à l'aide d'un synthétiseur automatique (Modèle 433A, APPLIED BIOSYSTEMS). La N-α-Fmoc-L-Lys(Biotine)-OH a été fournie par NEOSYSTEM (groupe SNPE). Les dérivés N-α-Fmoc-L d'acides aminés, la résine 4-hydroxyméthylphényloxy et les réactifs utilisés pour la synthèse peptidique ont été fournis par PERKIN ELMER LIFE SCIENCES. Les chaînes peptidiques ont été assemblées de façon séquentielle sur 0,25 meq de résine hydroxyméthylphényloxy (1 % de réticulation ; 0,89 meq de groupe aminé/g), à l'aide de 1 mmol de dérivés N-α-Fmoc-L d'acides aminés. Les groupes protecteurs des chaînes latérales sont les suivants : trityl pour les résidus de cystéine et d'asparagine ; *tert*-butyl pour les résidus de sérine, de thréonine, d'acide glutamique et d'acide aspartique ; pentaméthylchromane pour les résidus d'arginine, et *tert-*butyloxycarbonyle pour les résidus de lysine.

Les groupes N-α-aminés ont été déprotégés par traitement avec de la pipéridine/N-méthylpyrrolidone (18 et 20 % v/v), pendant respectivement 3 et 8 minutes. Les dérivés Fmoc d'acide aminé ont été couplés (20 min), comme leurs esters actifs d'hydroxybenzotriazole, dans de la N-méthylpyrrolidone (en excès d'un facteur 4). Après l'assemblage de la chaîne peptidique, la résine contenant le peptide (environ 1,8 mg) a été traitée pendant 2 à 3 heures à température ambiante, en agitation continue, avec un mélange d'acide trifluoroacétique/H₂O/thioanisol/éthanedithiol (88:5/5/2, v/v) en présence de cristaux de phénol (2,25 g). Le mélange peptidique a ensuite été filtré, et le filtrat a été précipité par addition d'éther de butylméthyle froid. Le peptide brut a été culoté par centrifugation (3 000 g pendant 10 min), et le surnageant a été éliminé. Le peptide réduit a ensuite été dissout dans du tampon Tris-HCl 200 mM, pH 8,3, à une concentration finale de 2,5 mM et mélangé à l'air libre, pendant 50 à 72 h, à température ambiante, afin de permettre son oxydation et son repliement.

Les produits ont ensuite été purifiés à homogénéité, par une première étape de chromatographie liquide en phase inverse, à haute pression (HPLC ; colonne C18 Aquapore ODS, 20 µm, 250x10 mm, Perkin Elmer Life Sciences), au moyen d'un gradient linéaire de 60 min d'acide trifluoroacétique (0,08 % v/v), 0 à 30 % d'acétonitrile dans 0,1 % (v/v) d'acide trifluoroacétique dans H₂O, à un débit de 6 ml/min (λ = 230 nm). Une seconde étape de purification de la maurocalcine, des peptides issus de la maurocalcine selon l'invention, et de leurs dérivés biotinylés a été réalisée par chromatographie échangeuse d'ion, sur une matrice de carboxyméthylcellulose, à l'aide de tampons phosphate 10 mM (tampon A) et 590 mM (tampon B), pH 9,0 (gradient linéaire de 0 à 60 % de tampon B à un débit de 1ml/min, pendant 1 heure). L'homogénéité et l'identité de la maurocalcine, des peptides issus de la maurocalcine selon la présente invention et de leurs dérivés biotinylés a été déterminée par : (i) chromatographie liquide haute-pression en phase inverse analytique C18 ( C18 Li-Chrospher, 5 µm, 4x20 mm, Merck), à l'aide d'un gradient linéaire de 60 min d'acide trifluoroacétique 0,08 % v/v/0-60 % d'acétonitrile dans 0,1 % v/v d'acide trifluoroacétique/H₂O, à un débit de 1 ml/min ; (ii) analyse d'acides aminés par acidolyse (6N HCl/2 % (w/v) phénol, 20 h, à 118°C, sous azote) et (iii) détermination de masse par spectrométrie de masse (MALDI).

### b) Formation de complexes avec la streptavidine couplée à la cyanine 3

La streptavidine-cyanine 3 et la streptavidine-cyanine 5 solubles (Strept-Cy³ et Strept-Cy⁵_{'} AMERSHAM) ont été mélangées avec 4 équivalents molaires de maurocalcine biotinylée (1 mM) ou de peptide issu de la maurocalcine biotinylé, en tampon phosphate (PBS en mM : NaCl 136, Na₂HPO₄ 1,47, KCl 2,6, CaCl₂ 1, MgCl₂ 0,5, pH 7,2), pendant 2 heures à 37°C et à l'obscurité,

### c) Cultures cellulaires

La lignée L6 de cellules myogéniques de rat (clone C5, EACC) est cultivée dans du milieu DMEM supplémenté avec 15 % de sérum bovin fétal (LIFE TECHNOLOGIES) et 1 % de pénicilline-streptomycine (Invitrogen).

La différenciation de la lignée L6 a été induite par remplacement du milieu de culture par du milieu de différenciation (DMEM + 5 % sérum de cheval), lorsque les cellules deviennent confluentes.

Les neurones de la région CA1 de l'hippocampe sont préparés à partir d'hippocampes de souris nouveaux-nés (1 à 2 jours post partum), disséqués, débarrassés des méninges et placés dans du tampon HBSS (INVITROGEN). Ensuite, ils sont incubés dans du milieu de dissociation (HBSS, 1 % pénicilline/streptomycine (GIBCO), 2 000 UI/ml DNase et 1 % (w/v) trypsine/EDTA), pendant 7 min, à 37°C. Après sédimentation, le surnageant est enlevé et le tissu est lavé avec du milieu HBSS contenant 1 % pénicilline/streptomycine. Le tissu est trituré doucement dans du milieu HBSS, 1 % pénicilline/streptomycine, 10 % sérum bovin fétal, 2 000 UI/ml DNase I, à l'aide d'une pipette en plastique, jusqu'à l'obtention d'une suspension homogène. Après centrifugation à 100 g pendant 1 min, le culot de cellules est remis en suspension dans du milieu Neurobasal/B27 (GIBCO) contenant 0,5 mM de L-glutamine et 1 % de pénicilline/streptomycine. Les cultures de cellules sont ensemencées à une densité de 10⁵ cellules/cm², dans des boîtes de culture préalablement traitées avec 20 µg/ml de poly-L-lysine, pendant 2 heures à 37°C. Après 2 jours de culture, du cytosine arabinoside (3 µM) est ajouté aux cultures pour limiter la prolifération des cellules non-neuronales, et 24 heures plus tard, la moitié du milieu est changée. Ensuite, le milieu de culture est changé tous les 2 jours.

Les cellules embryonnaires de rein humain (lignée HEK 293, ATCC) sont divisées avant confluence, par traitement à la trypsine (1 %) et sont maintenues dans du milieu DMEM (INVITROGEN) contenant 10 % de sérum de veau foetal inactivé (INVITROGEN) et 1 % de pénicilline/streptomycine (INVITROGEN), dans un incubateur à CO₂ (5 %). Le milieu de culture est changé tous les 2 jours.

### d) Immunocytochimie

### d₁) cellules fixées

Les cellules ont été incubées avec la maurocalcine et les peptides dérivés, biotinylés et complexés à la streptavidine-cyanine 3 ou à la streptavidine-cyanine 5, à la concentration finale de 100 nM dans du PBS, à l'obscurité et à température ambiante, pendant 30 minutes à 1 heure. Après 3 lavages en PBS, les cellules sont fixées à température ambiante, dans une solution de paraformaldéhyde (4 %), pendant 10 min, à l'obscurité, lavées en PBS et incubées pendant une heure avec de la concanavaline conjuguée au FITC (MOLECULAR PROBES, 5 µg/ml) pour marquer la membrane plasmique et de l'iodure de TO-PRO-3 (MOLECULAR PROBES, 1 µM), pour marquer le noyau. Pour marquer le cytosquelette, les cellules ont été fixées et perméabilisées avec du méthanol glacé, pendant 10 min, lavées deux fois en PBS et incubées avec un anticorps de souris anti-tubuline alpha (1:1000, SIGMA), pendant 2 heures. Après deux lavages en PBS, les cellules ont été incubées pendant une heure avec un anticorps secondaire anti-IgG de souris conjugué à l'Alexa 488 (1 :1000, MOLECULAR PROBES). Les cellules ont ensuite été montées dans du milieu de montage Vectashield (VECTOR LABORATORIES). Les préparations ont été observées en microscopie confocale à balayage laser, à l'aide d'un système de commande LEICA TCS-SP2. Les fluorescences de l'Alexa-488 et du Cy3 ou de l'iodure de propidium (IP) ont été excitées de façon séquentielles puis enregistrées. La fluorescence Cy3 a été excitée à l'aide d'un faisceau de 543 nm d'un laser hélium-néon et l'émission de fluorescence a été enregistrée entre 554 nm et 625 nm. Les images ont été importées dans Adobe Photoshop 7.0.

### d₂) cellules vivantes

Les cellules vivantes ont été incubées avec la maurocalcine et les peptides dérivés, biotinylés et complexés à la streptavidine-cyanine 3, à la concentration finale de 100 nM dans du PBS, à température ambiante dans du milieu de culture fraîchement changé, sur le plateau d'un microscope droit (Eclipse 600 FN, Nikon, équipé d'un objectif à immersion dans l'eau (x 40) ; ouverture 0,8) et d'une tête confocale (PCM 2000, Nikon), ou bien avec un microscope LEICA TCS-SP2, équipé d'un objectif (x 100), selon le mode « XYZt ».

La cinétique de pénétration de la maurocalcine et des peptides dérivés, biotinylés et complexés à la streptavidine-Cy3 a été initiée par l'injection de 100 nM du complexe dans le milieu de culture. La fluorescence Cy3 a été excitée à l'aide de la longueur d'onde de 543 nm d'un laser à argon. L'émission de la lumière a été filtrée (filtre de 595 ± 35 nm). Les images ont été enregistrées et analysées à l'aide du logiciel EZ2000 (Nikon). L'analyse quantitative de la fluorescence relative a été effectuée sur le signal global de l'ensemble des cellules, à l'aide d'un logiciel approprié (LEICA).

### e) Cytométrie de flux

Les cellules ont été incubées avec la maurocalcine et les peptides dérivés, biotinylés et complexés à la streptavidine-cyanine 5 (complexes MCa_{b}-Strept-Cy⁵), à la concentration finale de 100 nM dans du PBS, à l'obscurité et à température ambiante, pendant 1 heure. Après 2 lavages en PBS (PBS en mM : 0,15 NaCl, 6,84 Na₂HPO₄, 3,16 NaH₂PO₄, pH 7,2), les cellules ont été traitées avec de la trypsine (1 mg/ml, INVITROGEN), pendant 10 min à 37°C ; pour éliminer les complexes extracellulaires et les complexes liés à la membrane plasmique. Après l'incubation avec la trypsine, la suspension cellulaire a été centrifugée à 500 g et les cellules ont été remises en suspension dans du PBS contenant 1 µg/ml d'iodure de propidium (SIGMA). Pour les expériences n'incluant pas d'étape de traitement à l'iodure de propidium (courbe dose-réponse), le complexe MCa_{b}-Strept-Cy³ est utilisé à la place du complexe MCa_{b}-Strept-Cy⁵. L'analyse a été réalisée sur des cellules vivantes, à l'aide d'un cytomètre de flux (FACScalibur, BECTON DICKINSON). Les données ont été enregistrées et analysées à l'aide d'un logiciel approprié (CellQuest, BD BIOSCIENCES). Les cellules vivantes ont été triés selon leur taille et leur granulosité (*forward*/*side scattering*), sur un total de 10 000 évènements.

### 2) Résultats

### a) Analyse de la pénétration

Des variants de la maurocalcine (SEQ ID NO : 2 à 8 et 12 à 23), ainsi que deux chimères entre la maurocalcine et, soit l'impératoxine (SEQ ID NO : 24), soit l'opicalcine (SEQ ID NO : 25) ont été synthétisées :
- GDCLPHLKRCKADNDCCSKKCKRAGTNIEKRCR (SEQ ID NO : 24) et
- GDCLPHLKRCKENNDCCSKKCKRAGTNIEKRCR (SEQ ID NO : 25).
Chacune des chimères comprend en outre la substitution en alanine, de l'arginine en position 24 de la séquence de la maurocalcine (R24A) ou en position 15 (X₁₅ = A) de la formule (I).

La capacité des peptides dérivés de la maurocalcine selon la présente invention à transduire différents types de cellules et à transporter des macromolécules est étudiée à l'aide de complexes entre des peptides biotinylés et de la streptavidine couplée à la cyanine-3. Les complexes maurocalcine biotinylée-streptavidine-Cy3 (MCa_{b}/Strept-Cy3) servent de contrôle.

Les neurones primaires d'hippocampe de rat, et les cellules des lignées HEK293 et L6 (avant et après différenciation), sont incubés pendant 30 min, à température ambiante, avec 100 nM ou 333 nM de peptide dérivé de maurocalcine, biotinylé et complexé à la streptavidine-Cy3 ou bien 100 nM ou 333 nM de MCa_{b}/Strept-Cy3, à titre de contrôle. Les cellules sont fixées et la fluorescence est observée en microscopie confocale.

Les résultats obtenus avec les complexes MCa_{b}/Strept-Cy3 (figure 2), valident l'approche expérimentale utilisée pour analyser la pénétration dans les cellules, des peptides dérivés de la maurocalcine, biotinylés et complexés à la streptavidine-Cy3. En effet, on observe un fort marquage uniforme de la membrane plasmique et du cytoplasme de toutes les cellules, alors que le noyau est faiblement marqué. En revanche, les cellules incubées en présence de 100 nM de maurocalcine non-biotinylée ou de concentration équivalente de Strept-Cy3 ne montrent aucun marquage démontrant que Strept-Cy3 seul, n'est pas capable de transduire les cellules. L'association de Strept-Cy3 avec la maurocalcine biotinylée est nécessaire pour que le complexe fluorescent rentre dans les cellules, démontrant le rôle actif de la maurocalcine dans ce processus. Ces résultats démontrent que la maurocalcine ne se comporte pas uniquement comme un peptide de pénétration cellulaire (CPP) mais que, comme les autres CPP, elle est également capable de transporter des protéines de haut poids moléculaire, dans les cellules (la streptavidine est 14,6 fois plus grosse que la maurocalcine). '

Les variants de la maurocalcine sont également capables de pénétrer dans les cellules et de transporter des substances dans ces cellules (figure 3).

L'analyse quantitative (figures 4 et 5) montre que la pénétration est non saturable, ce qui est cohérent avec un mécanisme de diffusion de la maurocalcine et des peptides dérivés. La pénétration de la maurocalcine et des peptides dérivés est très efficace, puisqu'ils pénètrent dans les cellules à des concentrations aussi faibles que 10 nM. Certains variants (L7A, par exemple ; figure 5) ont une activité de pénétration significativement augmentée par rapport à la maurocalcine (facteur 5 pour le variant L7A), alors que d'autres ont une pénétration comparable, voire légèrement inférieure à celle de la maurocalcine (variant R24A par exemple ; figure 5).

### b) Cinétique de pénétration

### b₁) Analyse sur une période d'une heure

L'analyse par microscopie confocale de la cinétique de fluorescence de cellules L6 vivantes non-différenciées, sur une période d'une heure, montre que la marquage apparaît dès 3 min après l'addition de MCa_{b}/Strept-Cy3 dans le milieu extracellulaire (100 nM) (Figure 6A). Trois régions d'intérêt, correspondant respectivement à la membrane plasmique (ROI-1), au cytoplasme (ROI-2) et au noyau (ROI-3), ont été définies. Le positionnement des différentes régions d'intérêt a été facilité par l'analyse de l'image de la lumière transmise par la cellule observée (figure 6B). L'évolution de l'intensité de fluorescence dans les différentes ROIs a été analysée en fonction du temps (figure 6C).

Au début de l'application de 100 nM de complexe MCa_{b}/Strept-Cy3 dans le milieu, l'intensité de fluorescence augmente dans tous les compartiments, mais avec des vitesses différentes. L'évolution la plus rapide et la plus forte se situe dans la membrane plasmique (ROI-1), avec un pic à 10 min. Une augmentation plus lente, mais importante de la fluorescence est également observée dans le compartiment cytoplasmique (ROI-2), alors qu'une augmentation beaucoup plus faible, mais supérieure au bruit de fond est observée dans le noyau (ROI-3).

La vitesse et l'intensité relative à laquelle la fluorescence augmente dans chaque compartiment sont cohérentes avec la direction de progression des complexes MCa_{b}/Strept-Cy3 dans la cellule, à savoir : de l'espace extracellulaire vers la membrane plasmique, de la membrane plasmique vers le cytoplasme, et puis du cytoplasme vers le noyau. Le signal enregistré dans le compartiment ROI-1 est vraisemblablement surestimé, du fait d'une contamination par la fluorescence de la sonde située dans le cytoplasme. En revanche, l'intensité relative de la fluorescence cytoplasmique devrait être plus précise. En outre, le passage du cytoplasme vers le noyau est très faible, indiquant que cette transition est beaucoup moins favorisée que les deux autres. L'évolution de la fluorescence a également été suivie après élimination des complexes MCa_{b}/Strept-Cy3 par lavage : 12 min d'incubation en présence de complexe MCa_{b}/Strept-Cy3 et 5 min de lavage (figure 6C).

Dans ces conditions, l'intensité de fluorescence de la région ROI-1 diminue, alors que celle de ROI-2 augmente et celle de ROI-3 reste constante. L'augmentation de la fluorescence dans le compartiment cytoplasmique est due à un transfert constant de complexes, de la membrane plasmique vers le cytoplasme.

### b₂) Analyse sur une période de 24 heures

La distribution cellulaire des complexes MCa_{b}/Strept-Cy5 dans les cellules HEK 293 a ensuite été analysée par microscopie confocale, après une heure d'incubation avec 333 nM de complexes (Figure 7A). Le marquage de la surface des cellules HEK 293 a été réalisé à l'aide de la concanavaline A alors que les noyaux des cellules ont été marqués à l'iodure de propidium (figure 7A). La comparaison de ces marquages avec celui des complexes MCa_{b}/Strept-Cy5 démontre clairement la présence des complexes, à la fois à la membrane plasmique et dans le cytoplasme. La distribution des complexes MCa_{b}/Strept-Cy5 a également été comparée à celle de la tubuline alpha, un marqueur du cytosquelette (figure 7B). Une absence de colocalisation est clairement démontrée, suggérant que le cytosquelette n'est pas impliqué dans la distribution des complexes. Ensuite, l'évolution de la distribution cellulaire des complexes MCa_{b}/Strept-Cy3 a été suivie au cours du temps. Après 2 heures d'incubation, le complexe est principalement présent dans le cytoplasme. Entre 4 heures et 24 heures d'incubation, il est principalement perinucléaire et colocalise avec le noyau. La cible biologique de la maurocalcine est le récepteur RyR qui est strictement localisé dans le cytoplasme. La localisation de MCa_{b}/Strept-Cy3 dans le noyau ne peut donc pas résulter de sa liaison à RyR1.

### EXEMPLE 2 : Analyse des mécanisés impliqués dans la pénétration cellulaire de la maurocalcine et des peptides dérivés.

### a) La pénétration de la maurocalcine et des peptides dérivés est indépendante de l'énergie et insensible aux inhibiteurs de l'endocytose

La contribution éventuelle d'un processus énergie-dépendant, dans l'entrée des complexes MCa_{b}/Strept-Cy3 dans les cellules a été étudiée (figure 8). L'effet d'une température décroissante sur l'entrée des complexes MCa_{b}/Strept-Cy3 montre que les complexes pénètrent encore dans les cellules à 4°C (figure 8B). A cette température, le complexe marque la membrane et le cytoplasme de façon similaire. L'effet des inhibiteurs de la pinocytose/endocytose a également été testé. A la fois, l'amiloride (3 mM, figure 8C) et la nystatine (50 µM, figure 8D) n'ont pas d'effet sur l'entrée du complexe dans les cellules ou sur sa distribution relative sur la membrane plasmique et dans le cytoplasme, confirmant encore que le mécanisme principal d'entrée de la maurocalcine dans la cellule n'est pas dépendant de l'énergie.

### b) La maurocalcine et les peptides dérivés diffusent au travers des membranes

### b₁) protocole expérimental

L'analyse est effectuée comme décrit à l'exemple 1. Pour les expériences de traitement à l'héparine, les complexes ont été préparés dans une solution d'héparine (héparine sodée d'intestin de bovin, SIGMA) et les cellules ont été lavées deux fois avec la solution d'héparine, avant et après l'incubation avec les complexes préparés dans la même solution d'héparine.

### b₂) résultats

La pénétration cellulaire a été analysée par cytométrie de flux, sur des cellules incubées avec les complexes MCa_{b}/Strept-Cy3, puis traitées à la trypsine, de façon à éliminer les complexes liés à la membrane plasmique, par l'intermédiaire de lipides, de récepteurs spécifiques ou de glycosaminoglycane héparane sulfate (HPSG). Il a été montré que cette méthode permettait effectivement d'étudier l'entrée dans les cellules des peptides de pénétration (Richard et al., J. Biol. Chem., 2003, 278, 585-590).

La figure 9A montre que l'héparine réduit la pénétration de la maurocalcine dans les cellules, en gênant son interaction avec le glycosaminoglycane héparane sulfate ou en altérant ces propriétés d'interaction avec les lipides chargés négativement, e, se liant à sa face basique. La figure 9A montre également que la fraction de MCa_{b}/Strept-Cy3 qui est associée à la surface externe de la membrane plasmique, notamment par fixation sur l'héparane sulfate est minime puisque l'effet du traitement à la trypsine sur la pénétration cellulaire est faible (comparer panneau de gauche et panneau de droite). Ces résultats indiquent que la pénétration de la maurocalcine et des peptides dérivés ne nécessite pas de transporteur ou d'héparane sulfate et n'implique donc pas de mécanisme d'endocytose.

La figure 4 montre également que la pénétration des complexes MCa_{b}/Strept-Cy3 n'est pas saturable, ce qui est compatible avec un mécanisme de diffusion de la maurocalcine et des peptides dérivés.

En outre, la figure 9B montre une absence d'incorporation d'iodure de propidium par les cellules vivantes incubées en présence de complexe MCa_{b}/Strept-Cy5 (concentration du complexe 1 µM) ; cette absence signe une absence notable de toxicité cellulaire de la maurocalcine en présence de streptavidine-CyS.

### c) La pénétration de la maurocalcine et des peptides dérivés est sensible au potentiel de membrane

### c₁) protocole expérimental

Les complexes MCa_{b}-Strept-Cy⁵ ont été préparés dans des solutions contenant différents rapports Nacl/KCl (composition en mM : Nacl 145 à 5, KCl 5 à 145, CaCl₂ 2,5, MgCl₂ 1,2, glucose 10, HEPES 10, pH 7,4).

Les cellules ont été lavées deux fois avec la solution NaCl/KCl utilisée pour la préparation des complexes, puis incubées avec la maurocalcine et les peptides dérivés, biotinylés et complexés à la streptavidine-cyanine 3 (complexes MCa_{b}-Strept-Cy³), à la concentration finale de 100 nM dans la même solution NaCl/KCl, à l'obscurité et à température ambiante, pendant 1 heure. Après 2 lavages dans le même tampon NaCl/KCl, la fluorescence de la cyanine 3 a été analysée sur des cellules vivantes, à l'aide d'un cytomètre de flux (FACScalibur, BECTON DICKINSON). Les données ont été enregistrées et analysées à l'aide d'un logiciel approprié (CellQuest, BD BIOSCIENCES). Les cellules vivantes ont été triées selon leur taille et leur granulosité (*forward*/*side scattering*), sur un total de 10 000 évènements.

### c₂) résultats

La dépolarisation des cellules par l'augmentation de la concentration en KCl extracellulaire limite la pénétration de la maurocalcine d'environ 50 % (figure 10A et 10B). L'analyse quantitative (figure 10B) montre que la dépolarisation de la membrane induit une décroissance linéaire de l'intensité de fluorescence. Ce résultat indique que le potentiel de membrane agit comme un moteur de pénétration de la molécule basique qu'est la maurocalcine.

### d) Interaction de la maurocalcine avec les lipides

### d₁) protocole expérimental

Le disialoganglioside NeuAcα2-BNeuAca2-3Galb1-aGlcb1-Cer (GD3) et le Dipalmitoylphosphatidylcholine (DPPC) sont fournis respectivement par MATREYA INC et SIGMA.

La pression de surface a été mesurée à l'aide d'un microtensiomètre automatique (µTHROUGH SX, KIBRON INC.). L'appareil permet l'enregistrement des cinétiques d'interaction d'un ligand avec un film monomoléculaire en utilisant une série de sondes en Téflon appropriées. Toutes les expériences sont effectuées dans une atmosphère contrôlée à 20 ± 1. Les films moléculaires des lipides indiqués ont été déposées sur une phase acqueuse d'eau ultrapure (800 µl), à partir de hexane : chloroforme : ethanol (11:5:4, v/v/v), comme décrit précédemment (Mahfoud et al., J. Biol. Chem., 2002, 277, 11292-11296). Après l'étalement du film, un lapse de temps minimum de 5 min a été respecté, afin de permettre l'évaporation du solvant. Pour mesurer l'interaction de MCa avec les monocouches de lipides, différentes concentrations de peptides ont été injectées dans le film monomoléculaire avec une seringue Hamilton de 10 ml et l'augmentation de pression résultante, produite par l'incorporation du peptide a été enregistrée jusqu'à ce que l'équilibre soit atteint (augmentation maximale de pression de surface généralement atteinte après une incubation de 100 à 150 min). Pour l'interaction dose dépendante entre MCa et GD3, les films monomoléculaires de GD3 ont été préparés à une pression de surface initiale (pᵢ) de 10 mN.m⁻¹. Les résultats ont été analysés avec un logiciel Filmware 2.5 (KIBRON INC.). L'exactitude du système pour la mesure des pressions de surface était de 0,25 mN.m⁻¹ dans les conditions expérimentales.

### d₂) résultats

Afin de déterminer si la maurocalcine est capable d'interagir avec des lipides membranaires spécifiques, des films monomoléculaires de DPPC et de ganglioside GD3 ont été étalés à l'interface air-eau. La maurocalcine a ensuite été ajoutée dans la phase aqueuse à la concentration de 1 uM. Les variations de pressions de surface du film ont été enregistrées en continu, en fonction du temps (figure 11A). Les résultats suggèrent que la maurocalcine est capable de pénétrer au travers de la monocouche de GD3, comme le montre l'augmentation importante de la pression de surface du film de GD3. En revanche, la maurocalcine ne produit aucun changement significatif de la pression de surface du film de DPPC, indiquant que la maurocalcine ne reconnaît pas ce glycérophospholipide. La figure 11B montre que l'interaction de la maurocalcine avec GD3 est dose-dépendante. L'interaction est détectable à des concentrations de 100 nM de maurocalcine et atteint un maximum à 750 nM. La concentration produisant 50 % de l'effet maximum est de 500 nM.

Ces résultats indiquent que le site initial d'interaction de la maurocalcine (MCa) avec les membranes plasmiques est localisé dans des domaines enrichis en gangliosides. La représentation du potentiel électrostatique de surface montre que la maurocalcine possède une face basique impliquant G₁ et toutes les lysines et une face hydrophobe et indique que la maurocalcine est une molécule très chargée possédant un moment dipolaire important (figure 1B). En conséquence, il pourrait être envisagé que les interactions GD3/MCa neutralisent la face basique de MCa et favorisent l'interaction de sa face hydrophobe avec la partie interne de la membrane. GD3 pourrait transitoirement transloquer de la face externe de la membrane vers la face interne pour libérer MCa qui pourrait alors établir de nouvelles interactions électrostatiques avec d'autres lipides chargés négativement ou des protéines, en raison d'un environnement plus riche en charges négatives.

### EXEMPLE 3: Analyse de l'effet des complexes peptides dérivés de MCa biotinylés/Strept-Cy3 sur l'activité biologique du récepteur RyR1.

### 1) Matériels et méthodes

### a) Préparation des vésicules lourdes du réticulum sarcoplasmique

Les vésicules lourdes du réticulum sarcoplasmique sont préparées selon la méthode de Kim et al. (J. Biol. Chem., 1983, 258, 9662-9668) modifiée, comme décrit dans Marty et al. (J. Biol. Chem., 2000, 275, 8206-8212). La concentration en protéine est mesurée par la méthode de Biuret.

### b) Test de liaison à la ryanodine tritiée

Les vésicules lourdes du réticulum sarcoplasmique (1 mg/ml) sont incubées à 37°C, pendant 2 heures et 30 min, dans du tampon de réaction comprenant 5 nM [³H]-ryanodine, 150 mM NaCl, 2 mM EGTA, 2 mM Ca²⁺ (*p*Ca=5) et 20 mM Hepes, pH 7,4. La maurocalcine, les peptides issus de la maurocalcine et leurs dérivés biotinylés sont ajoutés avant l'addition des vésicules lourdes du réticulum sarcoplasmique. La [³H]-ryanodine liée aux vésicules sarcoplasmiques lourdes est mesurée par filtration au travers de filtres Whatmann GF/B, suivi de trois lavages avec 5 ml de tampon de lavage glacé (150 mM NaCl, 20 mM Hepes, pH 7,4. La [³H]-ryanodine retenue sur les filtres est mesurée par scintillation liquide. La liaison non-spécifique est mesurée en présence de ryanodine marquée (20 µM). Les résultats sont présentés sous la forme des moyennes ± écart-type. Chaque expérience est réalisée en triplicat et répétée au moins deux fois.

### c) Mesure du relargage de Ca²⁺

Le relargage de Ca²⁺ par les vésicules lourdes du réticulum sarcoplasmique a été mesuré à l'aide d'un colorant sensible au Ca²⁺, l'antipyrylazo III. L'absorbance a été mesurée à 710 nm, à l'aide d'un spectrophotomètre à faisceau de diode (MOS-200, Optical System, BIOLOGIC). Les vésicules lourdes du réticulum sarcoplasmique (50 µg) ont été activement chargées de Ca²⁺, à 37°C, dans 2 ml de tampon contenant 100 mM KCl, 7,5 mM pyrophosphate de sodium, 20 mM MOPS, pH 7,0, additionné de 250 µM d'antipyrylazo III, 1 mM ATP/MgCl₂, 5 mM phosphocréatine et 12 µg/ml de créatine phosphokinase (Palade, J. Biol. Chem., 1987, 262, 6142-6148).

Le chargement du calcium commence par des additions séquentielles de 50 µM et 20 µM CaCl₂.

Dans ces conditions de chargement, aucun relargage de calcium induit par le calcium, n'interfère avec les observations. A la fin de chaque expérience, le Ca²⁺ restant dans les vésicules est mesuré par addition de 4 µM de ionophore de Ca²⁺, A231287 (Sigma), et le signal d'absorbance est calibré par deux additions consécutives de 20 µM CaCl₂.

### 2) Résultats

La figure 12 montre que les complexes MCa_{b}/Strept-Cy3 conservent la capacité de stimuler la liaison de la [³H]-ryanodine sur les vésicules lourdes du réticulum sarcoplasmique (panneau A) et d'induire le relargage de Ca²⁺ à partir de ces vésicules (panneau B).

La cinétique plus lente de relargage du Ca²⁺, induite par le complexe MCa_{b}/Strept-Cy3, par comparaison avec la maurocalcine seule, est indicatrice d'une efficacité légèrement réduite (figure 12B). Cette différence provient probablement d'une accessibilité réduite du complexe MCa_{b}/Strept-Cy3 au site de liaison de la maurocalcine active, sur le récepteur RyR1, en raison de l'encombrement dû au Strept-Cy3 (PM de la streptavidine de 60 000 Da par comparaison à 4 108 Da pour la MCa_{b}).

Les résultats obtenus avec le complexe MCa_{b}/Strept-Cy3 valident l'approche expérimentale utilisée pour identifier des peptides issus de la maurocalcine, dépourvus d'effet pharmacologique sur le récepteur RyR1.

La figure 13 montre que parmi les variants de la maurocalcine capables de pénétrer dans les cellules et de transporter des substances d'intérêt, le mutant L7A est moins actif sur le récepteur RyR1, MCa_{b}/Strept-Cy3 alors que le mutant R24A est inactif.

### EXEMPLE 4 : Utilisation de la maurocalcine ou de peptides dérivés pour la pénétration cellulaire de nanoparticules.

La maurocalcine biotinylée a été couplée à des nanoparticules conjuguées à la streptavidine (Qdot®, QUANTUMDOT CORPORATION), selon le protocole recommandé par le fournisseur. Des nanoparticules couplées à la streptavidine seule (Qdot Streptavidin Conjugate, QUANTUMDOT CORPORATION) ont été utilisées comme contrôle. Les nanoparticules ont un diamètre de 10 à 15 nM et sont couplées chacune à 5 à 7 molécules de streptavidine. Des cultures de cellules HEK293 ont été incubées pendant 1 heure avec 100 nM de maurocalcine couplée à des nanoparticules (Qdot®, QUANTUMDOT CORPORATION) ou des nanoparticules couplées à la streptavidine seule, puis les cellules ont été fixées et analysées en microscopie confocale, comme décrit à l'exemple 1. La figure 14 montre que la maurocalcine permet la pénétration cellulaire des nanoparticules.

## Revendications

1. Utilisation d'un vecteur peptidique pour l'adressage intracellulaire d'une substance d'intérêt, **caractérisée en ce que** ledit vecteur est constitué par un peptide dérivé de la maurocalcine qui est différent de la maurocalcine et qui répond à la séquence (I) suivante :
Z-X)-X₂-X₃-X₄-X₅-X₆-X₇-X₈-X₉-X₁₀-X₁₁-X₁₂-X₁₃₋X₁₄-X₁₅-X₁₆-X₁₇-Z' (I),
dans laquelle :
- X1 et X₁₇ représentent chacun une cystéine,
- X₄, X₅, X₇ et X₈ représentent chacun une lysine ou une arginine,
- X₂, X₃, X₆ et X₉ à X₁₆ représentent chacun un acide aminé, pourvu que X₁₅ et/ou X₁₆ représentent une lysine ou une arginine, et
- Z et/ou Z' sont absents ou représentent chacun une séquence de 1 à 35 acides aminés.

2. Utilisation selon la revendication 1, **caractérisée en ce que** X₂ et/ou X₆ représentent une cystéine.

3. Utilisation selon la revendication 1 ou la revendication 2, **caractérisée en ce que** X₉ est différent d'une arginine et d'une lysine.

4. Utilisation selon l'une quelconque des revendications 1 à 3, **caractérisée en ce que** :
- X₃ représente S ou G,
- X₉ représente R ou est différent de R et K,
- X₁₁ représente T, S ou A,
- X₁₃ représente un acide aminé hydrophobe sélectionné parmi: A, V, L, I, P, W, F et M, et/ou
- X₁₀, X₁₂ et/ou X₁₄, représentent un acide aminé sélectionné dans le groupe constitué par A, C, D, E, F, G, K, L, M, P et N.

5. Utilisation selon l'une quelconque des revendications 1 à 4, **caractérisée en ce que** Z' représente une arginine ou une lysine.

6. Utilisation selon l'une quelconque des revendications 1 à 5, **caractérisée en ce que** Z répond à la séquence (II) suivante :
Z₁-Z₂-Z₃-Z₄-Z₅-Z₆-Z₇-Z₈-Z₉-Z₁₀-Z₁₁-Z₁₂-Z₁₃-Z₁₄-Z₁₅-Z₁₆-Z₁₇YZ 1₈-Z₁₉-Z₂₀-Z₂₁-Z₂₂-Z₂₃-Z₂₄-Z₂₅-Z₂₆-Z₂₇-Z₂₈-Z₂₉-Z₃₀-Z₃₁-Z₃₂-Z₃₃-Z₃₄-Z₃₅ (II),
dans laquelle : Z₁ à Z₃₅ représentent chacun un acide aminé ou sont absents, Z₃₁ ou Z₃₄ étant absents et Z₂₉, Z₃₀, Z₃₂, Z₃₃ et Z₃₅ étant toujours présents.

7. Utilisation selon la revendication 6, **caractérisée en ce que** Z₁ à Z₂₈ sont absents ou bien Z₁₉ à Z₂₈, Z₂₀ à Z₂₈, Z₂₁ à Z₂₈ ou Z₂₇ et Z₂₈ sont présents et Z₂₂ est absent.

8. Utilisation selon la revendication 6 ou la revendication 7, **caractérisée en ce que** Z₂₁ et/ou Z₂₉ représentent une cystéine.

9. Utilisation selon l'une quelconque des revendications 6 à 8, **caractérisée en ce que** lorsque Z₂₁ représente une cystéine, alors X₂ représente également une cystéine, et lorsque Z₂₉ représente une cystéine, alors X₆ représente également une cystéine.

10. Utilisation selon la revendication 9, **caractérisée en ce que** X₂, X₆, Z₂₁ et Z₂₉ représentent chacun une cystéine.

11. Utilisation selon l'une quelconque des revendications 6 à 10, **caractérisée en ce qu'**au moins un acide aminé choisi parmi Z₂₂ à Z₂₈ et Z₃₀ à Z₃₅ représente une lysine ou une arginine.

12. Utilisation selon la revendication 11, **caractérisée en ce que** Z₃₀ et/ou Z₃₄, Z₂₇ et/ou Z₂₈ représentent une arginine ou une lysine.

13. Utilisation selon l'une quelconque des revendications 10 à 12, **caractérisée en ce qu'**au moins trois acides aminés choisis parmi Z₂₂ à Z₂₈ et Z₃₀ à Z₃₅ représentent chacun une lysine ou une arginine.

14. Utilisation selon la revendication 13, **caractérisée en ce que** Z₂₇ et Z₃₀ représentent chacun une lysine et Z₂₈ ou Z₃₄ représentent une lysine ou une arginine.

15. Utilisation selon l'une quelconque des revendications 6 à 14, **caractérisée en ce que** Z₃₂, Z₃₃, et/ou Z₃₅, et éventuellement Z₁₉, Z₂₀, Z₂₃, Z₂₄, Z₂₅, Z₂₆, Z₂₈, Z₃₁, Z₃₄, et/ou Z'représentent un acide aminé sélectionné dans le groupe constitué par : A, C, D, E, F, G, K, L, M, P et N et H.

16. Utilisation selon l'une quelconque des revendications 1 à 15, **caractérisée en ce que** ledit peptide de séquence (I) est sélectionné dans le groupe constitué par les séquences SEQ ID NO : 2 et 7 à 25.

17. Utilisation selon l'une quelconque des revendications 1 à 16, **caractérisée en ce que** ledit peptide de séquence (I) est constitué d'acides aminés D.

18. Utilisation selon l'une quelconque des revendications 1 à 17, **caractérisée en ce que** ledit vecteur est un variant naturel ou synthétique de la maurocalcine.

19. Utilisation selon l'une quelconque des revendications 1 à 18, **caractérisé en ce que** ledit vecteur est couplé à la substance d'intérêt.

20. Utilisation selon l'une quelconque des revendications 1 à 19, **caractérisée en ce que** la séquence d'acides aminés du vecteur est fusionnée à la séquence peptidique ou polypeptidique d'un peptide ou d'une protéine hétérologue d'intérêt, de manière à former une protéine ou un peptide chimérique.

21. Utilisation selon l'une quelconque des revendications 1 à 20, **caractérisée en ce que** ledit vecteur est couplé à des particules.

22. Utilisation selon la revendication 21, **caractérisée en ce que** lesdites particules comprennent la substance d'intérêt.

23. Utilisation selon l'une quelconque des revendications 1 à 22, **caractérisée en ce que** ladite substance d'intérêt est une substance pharmacologiquement active dont la cible est intracellulaire.

24. Utilisation selon l'une quelconque des revendications 1 à 23, **caractérisée en ce que** ladite substance d'intérêt est un ligand d'un composant intracellulaire à détecter.

25. Utilisation selon la revendication 24, **caractérisée en ce que** ledit ligand est un anticorps ou un fragment fonctionnel d'anticorps, dirigé contre ledit composant intracellulaire.

26. Utilisation selon l'une quelconque des revendications 1 à 25, **caractérisée en ce que** ledit vecteur est couplé à un marqueur approprié, à des particules marquées ou bien à une substance marquée.

27. Composition comprenant une substance pharmacologiquement active dont la cible est intracellulaire et un vecteur tel que défini à l'une quelconque des revendications 1 à 23.

28. Composition utile comme réactif de détection intracellulaire, comprenant un ligand d'un composant intracellulaire à détecter et un vecteur tel que défini à l'une quelconque des revendications 1 à 22, et 24 à 26.

29. Composition selon la revendication 27, comme médicament.

30. Utilisation de la composition selon la revendication 27, pour la préparation d'un médicament, destiné au traitement d'une pathologie chez l'homme ou l'animal.

31. Composition selon la revendication 28, comme réactif de diagnostic.

32. Méthode *in vitro* de détection d'un composant intracellulaire, **caractérisée en ce qu'**elle comprend :
- la mise en contact d'un échantillon cellulaire avec un réactif de détection tel que défini à la revendication 28, et
- la détection d'un marquage intracellulaire, par tout moyen approprié.

33. Peptide tel que défini à l'une quelconque des revendications 1 à 18, à l'exception des peptides sélectionnés dans le groupe constitué par les séquences SEQ ID NO: 1 à 11, 26, 27 et STSRKLKS(PO₃H₂)QGTRRGKNRTPHKGVKRGCSKRKYRKSSLKSRKRCDDANRNFRSHL.

34. Peptide marqué dérivé du peptide selon la revendication 33.

35. Particules couplées au peptide de séquence (I) tel que défini à l'une quelconque des revendications 1 à 18.

36. Polynucléotide, **caractérisé en ce qu'**il code pour le peptide selon la revendication 33.

37. Vecteur recombinant comprenant le polynucléotide selon la revendication 36.

38. Cellules eucaryotes ou procaryotes modifiées par un polynucléotide selon la revendication 36 ou un vecteur recombinant selon la revendication 37.

39. Mammifère non-humain transgénique, **caractérisé en ce que** tout ou partie de ses cellules sont modifiées par un polynucléotide selon la revendication 36 ou un vecteur recombinant selon la revendication 37.

40. Plante transgénique, **caractérisée en ce que** tout ou partie de ses cellules sont modifiées par un polynucléotide selon la revendication 36 ou un vecteur recombinant selon la revendication 37.

## Claims

1. Use of a peptide vector for the intracellular addressing of a substance of interest, **characterized in that** said vector consists of a maurocalcine-derived peptide which is different from maurocalcine and which corresponds to the following sequence (I):
Z-x₁-X₂-X₃-X₄-X₅-X₆-X₇-X₈-X₉-X₁₀-X₁₁-X₁₂-X₁₃-X₁₄-X₁₅₋X₁₆-X₁₇-Z' (I),
in which:
- X₁ and X₁₇ each represent a cysteine,
- X₄, X₅, X₇ and X₈ each represent a lysine or an arginine,
- X₂, X₃, X₆ and X₉ to X₁₆ each represent an amino acid, provided that X₁₅ and/or X₁₆ represent a lysine or an arginine, and
- Z and/or Z' are absent or each represent a sequence of 1 to 35 amino acids.

2. Use according to claim 1, **characterized in that** X₂ and/or X₆ represent a cysteine.

3. Use according to claim 1 or claim 2, **characterized in that** X₉ is different from an arginine and from a lysine.

4. Use according to any one of claims 1 to 3, **characterized in that**:
- X₃ represents S or G,
- X₉ represents R or is different from R and K,
- X₁₁ represents T, S or A,
- X₁₃ represents a hydrophobic amino acid selected from: A, V, L, I, P, W, F and M, and/or,
- X₁₀, X₁₂ and/or X₁₄ represent an amino acid selected from the group consisting of A, C, D, E, F, G, K, L, M, P and N.

5. Use according to any one of claims 1 to 4, **characterized in that** Z' represents an arginine or a lysine.

6. Use according to any one of claims 1 to 5, **characterized in that** Z corresponds to the following sequence (II):
Z₁-Z₂-Z₃-Z₄-Z₅-Z₆-Z₇-Z₈-Z₉-Z₁₀-Z₁₁-Z₁₂-Z₁₃-Z₁₄-Z₁₅-Z₁₆-Z₁₇-Z₁₈-Z₁₉-Z₂₀-Z₂₁-Z₂₂-Z₂₃-Z₂₄-Z₂₅-Z₂₆-Z₂₇-Z₂₈-Z₂₉-Z₃₀-Z₃₁-Z₃₂-Z₃₃-Z₃₄-Z₃₅ (II),
in which: Z₁ to Z₃₅ each represent an amino acid or are absent, Z₃₁ or Z₃₄ being absent and Z₂₉, Z₃₀, Z₃₂, Z₃₃ and Z₃₅ always being present.

7. Use according to claim 6, **characterized in that** Z₁ to Z₂₈ are absent or else Z₁₉ to Z₂₈, Z₂₀ to Z₂₈, Z₂₁ to Z₂₈ or Z₂₇ and Z₂₈ are present and Z₂₂ is absent.

8. Use according to claim 6 or claim 7, **characterized in that** Z₂₁ and/or Z₂₉ represent a cysteine.

9. Use according to any one of claims 6 to 8, **characterized in that**, when Z₂₁ represents a cysteine, then X₂ also represents a cysteine, and when Z₂₉ represents a cysteine, then X₆ also represents a cysteine.

10. Use according to claim 9, **characterized in that** X₂, X₆, Z₂₁ and Z₂₉ each represent a cysteine.

11. Use according to any one of claims 6 to 10, **characterized in that** at least one amino acid chosen from Z₂₂ to Z₂₈ and Z₃₀ to Z₃₅ represents a lysine or an arginine.

12. Use according to claim 11, **characterized in that** Z₃₀ and/or Z₃₄, Z₂₇ and/or Z₂₈ represent an arginine or a lysine.

13. Use according to any one of claims 10 to 12, **characterized in that** at least three amino acids chosen from Z₂₂ to Z₂₈ and Z₃₀ to Z₃₅ each represent a lysine or an arginine.

14. Use according to claim 13, **characterized in that** Z₂₇ and Z₃₀ each represent a lysine and Z₂₈ or Z₃₄ represent a lysine or an arginine.

15. Use according to any one of claims 6 to 14, **characterized in that** Z₃₂, Z₃₃, and/or Z₃₅, and optionally Z₁₉, Z₂₀, Z₂₃, Z₂₄, Z₂₅, Z₂₆, Z₂₈, Z₃₁, Z₃₄, and/or Z' represent an amino acid selected from group the consisting of: A, C, D, E, F, G, K, L, M, P and N and H.

16. Use according to any one of claims 1 to 15, **characterized in that** said peptide of sequence (I) is selected from the group consisting of the sequences SEQ ID Nos. 2 and 7 to 25.

17. Use according to any one of claims 1 to 16, **characterized in that** said peptide of sequence (I) consists of D amino acids.

18. Use according to any one of claims 1 to 17, **characterized in that** said vector is a natural or synthetic variant of maurocalcine.

19. Use according to any one of claims 1 to 18, **characterized in that** said vector is coupled to the substance of interest.

20. Use according to any one of claims 1 to 19, **characterized in that** the amino acid sequence of the vector is fused to the peptide or polypeptide sequence of a heterologous peptide or protein of interest, so as to form a chimeric protein or peptide.

21. Use according to any one of claims 1 to 20, **characterized in that** said vector is coupled to particles.

22. Use according to claim 21, **characterized in that** said particles comprise the substance of interest.

23. Use according to any one of claims 1 to 22, **characterized in that** said substance of interest is a pharmacologically active substance whose target is intracellular.

24. Use according to any one of claims 1 to 23, **characterized in that** said substance of interest is a ligand of an intracellular component to be detected.

25. Use according to claim 24, **characterized in that** said ligand is an antibody or a functional fragment of an antibody directed against said intracellular component.

26. Use according to any one of claims 1 to 25, **characterized in that** said vector is coupled to an appropriate label, to labeled particles or else to a labeled substance.

27. Composition comprising a pharmacologically active substance whose target is intracellular and a vector as defined in any one of claims 1 to 23.

28. Composition that can be used as an intracellular detection reagent, comprising a ligand of an intracellular component to be detected and a vector as defined in any one of claims 1 to 22 and 24 to 26.

29. Composition according to claim 27, as a medicament.

30. Use of the composition according to claim 27, for the preparation of a medicament for use in the treatment of a pathology in humans or animals.

31. Composition according to claim 28, as a diagnostic reagent.

32. *In vitro* method of detecting an intracellular component, **characterized in that** it comprises:
- bringing a cell sample into contact with a detection reagent as defined in claim 28, and
- detecting an intracellular labeling, by any appropriate means.

33. Peptide as defined in any one of claims 1 to 18, with the exception of the peptides selected from the group consisting of the sequences SEQ ID Nos: 1 to 11, 26, 27 and STSRKLKS(PO₃H₂)QGTRRGKNRTPHKGVKRGCSKRKYRKSSLKSRKRCDDANRNFRSHL.

34. Labeled peptide derived from the peptide according to claim 33.

35. Particles coupled to the peptide of sequence (I) as defined in any one of claims 1 to 18.

36. Polynucleotide, **characterized in that** it encodes the peptide according to claim 33.

37. Recombinant vector comprising the polynucleotide according to claim 36.

38. Eukaryotic or prokaryotic cells modified with a polynucleotide according to claim 36 or a recombinant vector according to claim 37.

39. Transgenic nonhuman mammal, **characterized in that** all of part of its cells are modified with a polynucleotide according to claim 36 or a recombinant vector according to claim 37.

40. Transgenic plant, **characterized in that** all or part of its cells are modified with a polynucleotide according to claim 36 or a recombinant vector according to claim 37.

## Patentansprüche

1. Verwendung eines Peptidvektors für die intrazelluläre Targetierung einer Substanz von Interesse, **dadurch gekennzeichnet, dass** der Vektor durch ein Peptid gebildet wird, das von Maurocalcin abgeleitet ist, das sich von Maurocalcin unterscheidet und das der folgenden Sequenz (I) entspricht:
**Z-X**₁-**X**₂-**X**₃-**X**₄-**X**₅-**X**₆-**X**₇-**X**₈-**X**₉-**X**₁₀-**X**₁₁-**X**₁₂-**X**₁₃-**X**₁₄-**X**₁₅-**X**₁₆-**X**₁₇-**Z**' (I),
in der:
- X₁ und X₁₇ jeweils ein Cystein darstellen,
- X₄, X₅, X₇ und X₈ jeweils ein Lysin oder ein Arginin darstellen,
- X₂, X₃, X₆ und X₉ bis X₁₆ jeweils eine Aminosäure darstellen, mit der Maßgabe, dass X₁₅ und/oder X₁₆ ein Lysin oder ein Arginin darstellen, und
- Z und/oder Z' nicht vorhanden sind oder jeweils eine Sequenz von 1 bis 35 Aminosäuren darstellen.

2. Verwendung gemäß Anspruch 1, **dadurch gekennzeichnet, dass** X₂ und/oder X₆ ein Cystein darstellen.

3. Verwendung gemäß Anspruch 1 oder Anspruch 2, **dadurch gekennzeichnet, dass** X₉ von einem Arginin und einem Lysin verschieden ist.

4. Verwendung gemäß einem der Ansprüche 1 bis 3, **dadurch gekennzeichnet, dass**:
- X₃ S oder G darstellt,
- X₉ R darstellt oder von R und K verschieden ist,
- X₁₁ T, S oder A darstellt,
- X₁₃ eine hydrophobe Aminosäure darstellt, die ausgewählt ist aus: A, V, L, I, P, W, F und M, und/oder
- X₁₀, X₁₂ und/oder X₁₄ eine Aminosäure, ausgewählt aus der Gruppe, bestehend aus A, C, D, E, F, G, K, L, M, P und N, darstellen.

5. Verwendung gemäß einem der Ansprüche 1 bis 4, **dadurch gekennzeichnet, dass** Z' ein Arginin oder ein Lysin darstellt.

6. Verwendung gemäß einem der Ansprüche 1 bis 5, **dadurch gekennzeichnet, dass** Z der folgenden Sequenz (II) entspricht:
**Z₁-Z₂-Z₃-Z₄-Z₅-Z₆-Z₇-Z₈-Z₉-Z₁₀-Z₁₁-Z₁₂-Z₁₃-Z₁₄-Z₁₅-Z₁₆-Z₁₇-Z₁₈-Z₁₉-Z₂₀-Z₂₁-Z₂₂-Z₂₃-Z₂₄-Z₂₅-Z₂₆-Z₂₇-Z₂₈-Z₂₉-Z₃₀-Z₃₁-Z₃₂-Z₃₃-Z₃₄-Z₃₅** (II),
in der: Z₁ bis Z₃₅ jeweils eine Aminosäure darstellen oder nicht vorhanden sind, Z₃₁ oder Z₃₄ nicht vorhanden sind und Z₂₉, Z₃₀, Z₃₂, Z₃₃ und Z₃₅ immer vorhanden sind.

7. Verwendung gemäß Anspruch 6, **dadurch gekennzeichnet, dass** Z₁ bis Z₂₈ nicht vorhanden sind oder auch Z₁₉ bis Z₂₈, Z₂₀ bis Z₂₈, Z₂₁ bis Z₂₈ oder Z₂₇ und Z₂₈ vorhanden sind und Z₂₂ nicht vorhanden ist.

8. Verwendung gemäß Anspruch 6 oder Anspruch 7, **dadurch gekennzeichnet, dass** Z₂₁ und/oder Z₂₉ ein Cystein darstellen.

9. Verwendung gemäß einem der Ansprüche 6 bis 8, **dadurch gekennzeichnet, dass**, wenn Z₂₁ ein Cystein darstellt, dann X₂ auch ein Cystein darstellt, und wenn Z₂₉ ein Cystein darstellt, dann X₆ auch ein Cystein darstellt.

10. Verwendung gemäß Anspruch 9, **dadurch gekennzeichnet, dass** X₂, X₆, Z₂₁ und Z₂₉ jeweils ein Cystein darstellen.

11. Verwendung gemäß einem der Ansprüche 6 bis 10, **dadurch gekennzeichnet, dass** wenigstens eine Aminosäure, ausgewählt aus Z₂₂ bis Z₂₈ und Z₃₀ bis Z₃₅, ein Lysin oder ein Arginin darstellt.

12. Verwendung gemäß Anspruch 11, **dadurch gekennzeichnet, dass** Z₃₀ und/oder Z₃₄, Z₂₇ und/oder Z₂₈ ein Arginin oder ein Lysin darstellen.

13. Verwendung gemäß einem der Ansprüche 10 bis 12, **dadurch gekennzeichnet, dass** wenigstens drei Aminosäuren, ausgewählt aus Z₂₂ bis Z₂₈ und Z₃₀ bis Z₃₅, jeweils ein Lysin oder ein Arginin darstellen.

14. Verwendung gemäß Anspruch 13, **dadurch gekennzeichnet, dass** Z₂₇ und Z₃₀ jeweils ein Lysin darstellen und Z₂₈ oder Z₃₄ ein Lysin oder ein Arginin darstellt.

15. Verwendung gemäß einem der Ansprüche 6 bis 14, **dadurch gekennzeichnet, dass** Z₃₂, Z₃₃ und/oder Z₃₅ und gegebenenfalls Z₁₉, Z₂₀, Z₂₃, Z₂₄, Z₂₅, Z₂₆, Z₂₈, Z₃₁, Z₃₄ und/oder Z' eine Aminosäure darstellen, die aus der Gruppe, bestehend aus A, C, D, E, F, G, K, L, M, P und N und H, ausgewählt ist.

16. Verwendung gemäß einem der Ansprüche 1 bis 15, **dadurch gekennzeichnet, dass** die Peptidsequenz (I) aus der Gruppe, bestehend aus den Sequenzen SEQ ID NO:2 und 7 bis 25, ausgewählt ist.

17. Verwendung gemäß einem der Ansprüche 1 bis 16, **dadurch gekennzeichnet, dass** die Peptidsequenz (I) aus Aminosäuren D besteht.

18. Verwendung gemäß einem der Ansprüche 1 bis 17, **dadurch gekennzeichnet, dass** der Vektor eine natürliche oder synthetische Variante des Maurocalcin ist.

19. Verwendung gemäß einem der Ansprüche 1 bis 18, **dadurch gekennzeichnet, dass** der Vektor an die Substanz von Interesse gekoppelt ist.

20. Verwendung gemäß einem der Ansprüche 1 bis 19, **dadurch gekennzeichnet, dass** die Aminosäuresequenz des Vektors an die Peptid- oder Polypeptidsequenz eines heterologen Peptids oder Proteins von Interesse derart fusioniert ist, dass ein chimäres Protein oder Peptid gebildet wird.

21. Verwendung gemäß einem der Ansprüche 1 bis 20, **dadurch gekennzeichnet, dass** der Vektor an Partikel gebunden ist.

22. Verwendung gemäß Anspruch 21, **dadurch gekennzeichnet, dass** die Partikel die Substanz von Interesse umfassen.

23. Verwendung gemäß einem der Ansprüche 1 bis 22, **dadurch gekennzeichnet, dass** die Substanz von Interesse eine pharmakologisch aktive Substanz ist, deren Ziel intrazellulär ist.

24. Verwendung gemäß einem der Ansprüche 1 bis 23, **dadurch gekennzeichnet, dass** die Substanz von Interesse ein Ligand eines zu detektierenden intrazellulären Bestandteils ist.

25. Verwendung gemäß Anspruch 24, **dadurch gekennzeichnet, dass** der Ligand ein Antikörper oder ein funktionelles Antikörperfragment ist, der/das gegen den intrazellulären Bestandteil gerichtet ist.

26. Verwendung gemäß einem der Ansprüche 1 bis 25, **dadurch gekennzeichnet, dass** der Vektor an einen geeigneten Marker, an markierte Partikel oder auch an eine markierte Substanz gebunden ist.

27. Zusammensetzung, umfassend eine pharmakologisch aktive Substanz, deren Ziel intrazellulär ist, und einen Vektor, wie in einem der Ansprüche 1 bis 23 definiert.

28. Zusammensetzung, die als intrazelluläres Detektionsreagenz verwendbar ist, umfassend einen Liganden eines zu detektierenden intrazellulären Bestandteils und einen Vektor, wie in einem der Ansprüche 1 bis 22 und 24 bis 26 definiert.

29. Zusammensetzung gemäß Anspruch 27 als Medikament.

30. Verwendung der Zusammensetzung gemäß Anspruch 27 für die Herstellung eines Medikaments, das zur Behandlung einer Pathologie bei Mensch oder Tier bestimmt ist.

31. Zusammensetzung gemäß Anspruch 28 als Diagnosereagenz.

32. In-vitro-Verfahren zur Detektion eines intrazellulären Bestandteils, **dadurch gekennzeichnet, dass** es umfasst:
- Inkontaktbringen einer zellulären Probe mit einem Detektionsreagenz, wie in Anspruch 28 definiert, und
- Detektion einer intrazellulären Markierung durch jedes geeignete Mittel.

33. Peptid, wie es in einem der Ansprüche 1 bis 18 definiert ist, mit Ausnahme der Peptide, die aus der Gruppe, bestehend aus den Sequenzen SEQ ID NO: 1 bis 11, 26, 27 und STSRKLKS(PO₃H₂₎QGTRRGKNRTPHKGVKRGCSKRKYRKSSLKSRKRCDDANRNFRSHL, ausgewählt sind.

34. Markiertes Peptid, das von dem Peptid gemäß Anspruch 33 abgeleitet ist.

35. Partikel, die an die Peptidsequenz (I), wie in einem der Ansprüche 1 bis 18 definiert, gekoppelt sind.

36. Polynukleotid, **dadurch gekennzeichnet, dass** es das Peptid gemäß Anspruch 33 codiert.

37. Rekombinanter Vektor, der das Polynukleotid gemäß Anspruch 36 umfasst.

38. Eukaryoten- oder Prokaryotenzellen, die durch ein Polynukleotid gemäß Anspruch 36 oder einen rekombinanten Vektor gemäß Anspruch 37 modifiziert sind.

39. Nichtmenschliches transgenes Säugetier, **dadurch gekennzeichnet, dass** alle seine Zellen oder ein Teil seiner Zellen durch ein Polynukleotid gemäß Anspruch 36 oder einen rekombinanten Vektor gemäß Anspruch 37 modifiziert sind.

40. Transgene Pflanze, **dadurch gekennzeichnet, dass** alle ihre Zellen oder ein Teil ihrer Zellen durch ein Polynukleotid gemäß Anspruch 36 oder einen rekombinanten Vektor gemäß Anspruch 37 modifiziert sind.


### SEQUENCE LISTING

<110> COMMISSARIAT A L'ENERGIE ATOMIQUE INSTITUT NATIONAL DE LA SANTE ET DE LA RECHERCHE MEDICALE RONJAT, Michel DE WAARD, Michel
<120> PEPTIDES DERIVES DE LA MAUROCALCINE UTILISABLES COMME VECTEURS POUR L'ADRESSAGE INTRACELLULAIRE DE MOLECULES D'INTERET
<130> HLPclg263PCT123
<160> 29
<170> PatentIn version 3.3
<210> 1
   <211> 33
   <212> PRT
   <213> scorpio maurus
<400> 1
   Gly Asp Cys Leu Pro His Leu Lys Leu Cys Lys Glu Asn Lys Asp Cys
   1 5 10 15
   Cys Ser Lys Lys Cys Lys Arg Arg Gly Thr Asn Ile Glu Lys Arg Cys
   20 25 30
   Arg
<210> 2
   <211> 33
   <212> PRT
   <213> artificial sequence
<220>
   <223> mutant K8A de la maurocalcine
<400> 2
   Gly Asp Cys Leu Pro His Leu Ala Leu Cys Lys Glu Asn Lys Asp Cys
   1 5 10 15
   Cys Ser Lys Lys Cys Lys Arg Arg Gly Thr Asn Ile Glu Lys Arg Cys
   20 25 30
   Arg
<210> 3
   <211> 33
   <212> PRT
   <213> artificial sequence
<220>
   <223> mutant K19A de la maurocalcine
<400> 3
   Gly Asp Cys Leu Pro His Leu Lys Leu Cys Lys Glu Asn Lys Asp Cys
   1 5 10 15
   Cys Ser Ala Lys Cys Lys Arg Arg Gly Thr Asn Ile Glu Lys Arg Cys
   20 25 30
   Arg
<210> 4
   <211> 33
   <212> PRT
   <213> artificial sequence
<220>
   <223> mutant K20A de la maurocalcine
<400> 4
   Gly Asp Cys Leu Pro His Leu Lys Leu Cys Lys Glu Asn Lys Asp Cys
   1               5                   10                  15
   Cys Ser Lys Ala Cys Lys Arg Arg Gly Thr Asn Ile Glu Lys Arg Cys
               20                  25                  30
   Arg
<210> 5
   <211> 33
   <212> PRT
   <213> artificial sequence
<220>
   <223> mutant K22A de la maurocalcine
<400> 5
   Gly Asp Cys Leu Pro His Leu Lys Leu Cys Lys Glu Asn Lys Asp Cys
   1               5                   10                  15
   Cys Ser Lys Lys Cys Ala Arg Arg Gly Thr Asn Ile Glu Lys Arg Cys
               20                  25                  30
   Arg
<210> 6
   <211> 33
   <212> PRT
   <213> artificial sequence
<220>
   <223> mutant R23A de la maurocalcine
<400> 6
   Gly Asp Cys Leu Pro His Leu Lys Leu Cys Lys Glu Asn Lys Asp Cys
   1               5                   10                  15
   Cys Ser Lys Lys Cys Lys Ala Arg Gly Thr Asn Ile Glu Lys Arg Cys
               20                  25                  30
   Arg
<210> 7
   <211> 33
   <212> PRT
   <213> artificial sequence
<220>
   <223> mutant R24A de la maurocalcine
<400> 7
   Gly Asp Cys Leu Pro His Leu Lys Leu Cys Lys Glu Asn Lys Asp Cys
   1               5                   10                  15
   Cys Ser Lys Lys Cys Lys Arg Ala Gly Thr Asn Ile Glu Lys Arg Cys
               20                  25                  30
   Arg
<210> 8
   <211> 33
   <212> PRT
   <213> artificial sequence
<220>
   <223> mutant T26A de la maurocalcine
<400> 8
   Gly Asp Cys Leu Pro His Leu Lys Leu Cys Lys Glu Asn Lys Asp Cys
   1               5                   10                  15
   Cys Ser Lys Lys Cys Lys Arg Arg Gly Ala Asn Ile Glu Lys Arg Cys
               20                  25                  30
   Arg
<210> 9
   <211> 33
   <212> PRT
   <213> Pandinus imperator
<400> 9
   Gly Asp Cys Leu Pro His Leu Lys Arg Cys Lys Ala Asp Asn Asp Cys
   1               5                   10                  15
   Cys Gly Lys Lys Cys Lys Arg Arg Gly Thr Asn Ala Glu Lys Arg Cys
               20                  25                  30
   Arg
<210> 10
   <211> 33
   <212> PRT
   <213> opistophtalmus *carinatis*
<400> 10
   Gly Asp Cys Leu Pro His Leu Lys Arg Cys Lys Glu Asn Asn Asp Cys
   1               5                   10                  15
   Cys Ser Lys Lys Cys Lys Arg Arg Gly Thr Asn Pro Glu Lys Arg Cys
               20                  25                  30
   Arg
<210> 11
   <211> 33
   <212> PRT
   <213> opistophtalmus *carinatis*
<400> 11
   Gly Asp Cys Leu Pro His Leu Lys Arg Cys Lys Glu Asn Asn Asp Cys
   1               5                   10                  15
   Cys Ser Lys Lys Cys Lys Arg Arg Gly Ala Asn Pro Glu Lys Arg Cys
               20                  25                  30
   Arg
<210> 12
   <211> 33
   <212> PRT
   <213> artificial sequence
<220>
   <223> mutant D2A de la maurocalcine
<400> 12
   Gly Ala Cys Leu Pro His Leu Lys Leu Cys Lys Glu Asn Lys Asp Cys
   1               5                   10                  15
   Cys Ser Lys Lys Cys Lys Arg Arg Gly Thr Asn Ile Glu Lys Arg Cys
               20                  25                  30
   Arg
<210> 13
   <211> 33
   <212> PRT
   <213> artificial sequence
<220>
   <223> mutant L4A de la maurocalcine
<400> 13
   Gly Asp Cys Ala Pro His Leu Lys Leu Cys Lys Glu Asn Lys Asp Cys
   1 5 10 15
   Cys Ser Lys Lys Cys Lys Arg Arg Gly Thr Asn Ile Glu Lys Arg Cys
   20 25 30
   Arg
<210> 14
   <211> 33
   <212> PRT
   <213> artificial sequence
<220>
   <223> mutant P5A de la maurocalcine
<400> 14
   Gly Asp Cys Leu Ala His Leu Lys Leu Cys Lys Glu Asn Lys Asp Cys
   1 5 10 15
   Cys Ser Lys Lys Cys Lys Arg Arg Gly Thr Asn Ile Glu Lys Arg Cys
   20 25 30
   Arg
<210> 15
   <211> 33
   <212> PRT
   <213> artificial sequence
<220>
   <223> mutant H6A de la maurocalcine
<400> 15
   Gly Asp Cys Leu Pro Ala Leu Lys Leu Cys Lys Glu Asn Lys Asp Cys
   1 5 10 15
   Cys Ser Lys Lys Cys Lys Arg Arg Gly Thr Asn Ile Glu Lys Arg Cys
   20 25 30
   Arg
<210> 16
   <211> 33
   <212> PRT
   <213> artificial sequence
<220>
   <223> mutant L7A de la maurocalcine
<400> 16
   Gly Asp Cys Leu Pro His Ala Lys Leu Cys Lys Glu Asn Lys Asp Cys
   1 5 10 15
   Cys Ser Lys Lys Cys Lys Arg Arg Gly Thr Asn Ile Glu Lys Arg Cys
   20 25 30
   Arg
<210> 17
   <211> 33
   <212> PRT
   <213> artificial sequence
<220>
   <223> mutant L9A de la maurocalcine
<400> 17
   Gly Asp Cys Leu Pro His Leu Lys Ala Cys Lys Glu Asn Lys Asp Cys
   1 5 10 15
   Cys Ser Lys Lys Cys Lys Arg Arg Gly Thr Asn Ile Glu Lys Arg Cys
   20 25 30
   Arg
<210> 18
   <211> 33
   <212> PRT
   <213> artificial sequence
<220>
   <223> mutant E12A de la maurocalcine
<400> 18
   Gly Asp Cys Leu Pro His Leu Lys Leu Cys Lys Ala Asn Lys Asp Cys
   1 5 10 15
   Cys Ser Lys Lys Cys Lys Arg Arg Gly Thr Asn Ile Glu Lys Arg Cys
   20 25 30
   Arg
<210> 19
   <211> 33
   <212> PRT
   <213> artificial sequence
<220>
   <223> mutant N13A de la maurocalcine
<400> 19
   Gly Asp Cys Leu Pro His Leu Lys Leu Cys Lys Glu Ala Lys Asp Cys
   1               5                   10                  15
   Cys Ser Lys Lys Cys Lys Arg Arg Gly Thr Asn Ile Glu Lys Arg Cys
               20                  25                  30
   Arg
<210> 20
   <211> 33
   <212> PRT
   <213> artificial sequence
<220>
   <223> mutnt D15A de la maurocalcine
<400> 20
   Gly Asp Cys Leu Pro His Leu Lys Leu Cys Lys Glu Asn Lys Ala Cys
   1               5                   10                  15
   Cys Ser Lys Lys Cys Lys Arg Arg Gly Thr Asn Ile Glu Lys Arg Cys
               20                  25                  30
   Arg
<210> 21
   <211> 33
   <212> PRT
   <213> artificial sequence
<220>
   <223> mutant G25A de la maurocalcine
<400> 21
   Gly Asp Cys Leu Pro His Leu Lys Leu Cys Lys Glu Asn Lys Asp Cys
   1               5                   10                  15
   Cys Ser Lys Lys Cys Lys Arg Arg Ala Thr Asn Ile Glu Lys Arg Cys
               20                  25                  30
   Arg
<210> 22
   <211> 33
   <212> PRT
   <213> artificial sequence
<220>
   <223> mutant N27A de la maurocalcine
<400> 22
   Gly Asp Cys Leu Pro His Leu Lys Leu Cys Lys Glu Asn Lys Asp Cys
   1               5                   10                  15
   Cys Ser Lys Lys Cys Lys Arg Arg Gly Thr Ala Ile Glu Lys Arg Cys
               20                  25                  30
   Arg
<210> 23
   <211> 30
   <212> PRT
   <213> artificial sequence
<220>
   <223> mutant E29A de la maurocalcine
<400> 23
   Leu Pro His Leu Lys Leu Cys Lys Glu Asn Lys Asp Cys Cys Ser Lys
   1               5                   10                  15
   Lys Cys Lys Arg Arg Gly Thr Asn Ile Ala Lys Arg Cys Arg
               20                  25                  30
<210> 24
   <211> 33
   <212> PRT
   <213> artificial sequence
<220>
   <223> chimère maurocalcine/impératoxine A
<400> 24
   Gly Asp Cys Leu Pro His Leu Lys Arg Cys Lys Ala Asp Asn Asp Cys
   1               5                   10                  15
   Cys Ser Lys Lys Cys Lys Arg Ala Gly Thr Asn Ile Glu Lys Arg Cys
               20                  25                  30
   Arg
<210> 25
   <211> 33
   <212> PRT
   <213> artificial sequence
<220>
   <223> chimère maurocalcine/opicalcine 1
<400> 25
   Gly Asp Cys Leu Pro His Leu Lys Arg Cys Lys Glu Asn Asn Asp Cys
   1 5 10 15
   Cys Ser Lys Lys Cys Lys Arg Ala Gly Thr Asn Ile Glu Lys Arg Cys
   20 25 30
   Arg
<210> 26
   <211> 20
   <212> PRT
   <213> ARTIFICIAL SEQUENCE
<220>
   <223> peptide synthétique
<400> 26
   Cys Trp Cys Lys Lys Lys Lys Lys Lys Lys Lys Lys Lys Lys Lys Lys
   1 5 10 15
   Lys Lys Cys Lys
   20
<210> 27
   <211> 20
   <212> PRT
   <213> artificial sequence
<220>
   <223> peptide synthétique
<400> 27
   Cys Trp Cys Lys Lys Lys Cys Lys Lys Lys Cys Lys Lys Lys Cys Lys
   1 5 10 15
   Lys Lys Cys Lys
   20
<210> 28
   <211> 20
   <212> PRT
   <213> artificial sequence
<220>
   <223> peptide synthétique
<400> 28
   Cys Trp Lys Lys Lys Lys Lys Cys Lys Lys Lys Lys Lys Cys Lys Lys
   1               5                   10                  15
   Lys Lys Lys Cys
               20
<210> 29
   <211> 34
   <212> PRT
   <213> Momordica cochinchinensis
<400> 29
   Ser Gly Ser Asp Gly Gly Val Cys Pro Lys Ile Leu Lys Lys Cys Arg
   1               5                   10                  15
   Arg Asp Ser Asp Cys Pro Gly Ala Cys Ile Cys Arg Gly Asn Gly Tyr
               20                  25                  30
   Cys Gly